(19) Europäisches Patentamt
European Patent Office
Office européen des brevets

(11) EP 4 505 993 A1

(12) EUROPEAN PATENT APPLICATION
published in accordance with Art. 153(4) EPC

(43) Date of publication:
**12.02.2025 Bulletin 2025/07**

(21) Application number: **23781121.1**

(22) Date of filing: **01.04.2023**

(51) International Patent Classification (IPC):
***A61K 6/887*** *(2020.01)* ***A61K 6/16*** *(2020.01)*
***A61K 6/17*** *(2020.01)* ***A61K 6/62*** *(2020.01)*
***A61K 6/831*** *(2020.01)* ***A61K 6/84*** *(2020.01)*
***A61K 6/842*** *(2020.01)* ***A61K 6/893*** *(2020.01)*

(52) Cooperative Patent Classification (CPC):
**A61K 6/16; A61K 6/17; A61K 6/62; A61K 6/831; A61K 6/84; A61K 6/842; A61K 6/887; A61K 6/893**

(86) International application number:
**PCT/JP2023/013749**

(87) International publication number:
**WO 2023/191113 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.04.2022 JP 2022062164**

(71) Applicant: **Kuraray Noritake Dental Inc.**
**Kurashiki-shi, Okayama 710-0801 (JP)**

(72) Inventors:
- **MATSUURA, Ryo**
**Tainai-shi, Niigata 959-2653 (JP)**
- **NOJIRI, Yamato**
**Tokyo 100-0004 (JP)**

(74) Representative: **D Young & Co LLP**
**3 Noble Street**
**London EC2V 7BQ (GB)**

# (54) SELF-ADHESIVE COMPOSITE RESIN FOR DENTAL USE

(57) The present invention provides a self-adhesive dental composite resin that can achieve both favorable cavity sealing properties and good shade conformity when used for cavities in wedge-shaped defects. The present invention relates to a self-adhesive dental composite resin comprising a monomer (a) having an acidic group, a monomer (b) having no acidic group, a polymerization initiator (c), and a filler (d),
wherein the self-adhesive dental composite resin has a polymerization shrinkage stress of less than 10.0 MPa, and the degree of light diffusion D as indicated by the following formula [I] for the self-adhesive dental composite resin after curing is 0.10 or more and less than 3.00,

$$D = (I_{20}/\cos 20° + I_{70}/\cos 70°)/(2I_0) \quad [I].$$

(Description of the symbols in the formula is omitted.)

EP 4 505 993 A1

## Description

### TECHNICAL FIELD

The present invention relates to self-adhesive dental composite resins. More specifically, the present invention relates to a self-adhesive dental composite resin that excels in bond strength to tooth structure, cavity sealing properties, and shade conformity. The present invention also relates to a self-adhesive dental composite resin that can achieve both favorable cavity sealing properties and good shade conformity, particularly when used for cavities in wedge-shaped defects.

### BACKGROUND ART

Restorative filling materials such as filling composite resins and filling compomers, and crown restoration materials such as metal alloys, porcelain, and resin materials are typically used for the restoration of damaged tooth structures (enamel, dentin, and cementum) caused by caries and other dental defects. However, restorative filling materials and crown restoration materials (in this specification, these are also referred to collectively as "dental restoration materials") themselves do not generally possess adhesive properties to the tooth structure. Consequently, various adhesive systems with bonding agents have been used for the bonding of the tooth structure and dental restoration materials. An example of adhesive systems that are in common use is the acid etching (total etching) adhesive system, whereby the surfaces of tooth structure are etched with an acid etchant such as a phosphoric acid aqueous solution, and a bonding material, or an adhesive, is applied to bond a dental restoration material to the tooth structure.

The self-etching adhesive system, on the other hand, is an example of adhesive systems that do not use acid etchants. In the past, the mainstream in such adhesive systems has been a two-step process in which a self-etching primer containing an acidic monomer, a hydrophilic monomer, and water is first applied to surfaces of tooth structure, and a bonding material containing a crosslinkable monomer and a polymerization initiator is applied without rinsing the surfaces with water. Another type of self-etching adhesive system that has come to be commonly used in recent years is the single-step adhesive system, which uses a one-part dental adhesive (one-part bonding material) that serves as both a self-etching primer and a bonding material. The typical monomer components of the one-part bonding material are acidic monomers, hydrophilic monomers, and crosslinkable monomers, and the one-part bonding material typically uses water and hydrophilic volatile organic solvents.

Recent years have seen the development of self-adhesive dental composite resins possessing adhesive properties in the dental composite resins themselves, and some of the compositions that have already been put into practical use eliminate the use of bonding materials to reduce the number of steps in restorative treatment procedures (Patent Literatures 1 to 3). Generally, bonding material differs from dental composite resin (such as self-adhesive dental composite resins) in that the bonding material contains a solvent (such as water or organic solvents), and differs in filler content from dental composite resins.

Generally, self-adhesive dental composite resins present challenges concerning bond strength to tooth structure and cavity sealing properties, compared to bonding materials.

To address these challenges, for example, self-adhesive dental composite resins are disclosed that comprise a water-soluble photopolymerization initiator with a solubility in water of 10 g/L or more at 25°C, or an asymmetrical acrylamide·methacrylic acid ester compound, in order to increase the characteristic bond strength of bonding materials (Patent Literatures 4 and 5). Self-adhesive dental composite resins are also disclosed that comprise a dental composition containing polymer particles and having a specific degree of light diffusion, or a (meth)acrylic compound (A) with a defined average molecular weight and a defined average molecular weight per (meth)acryl group, in order to decrease the characteristic polymerization shrinkage of composite resins (Patent Literatures 6 and 7).

### CITATION LIST

#### Patent Literature

Patent Literature 1: JP 2004-529946 T
Patent Literature 2: JP 2017-105716 A
Patent Literature 3: JP 2018-065831 A
Patent Literature 4: WO2019/044815
Patent Literature 5: WO2015/190101
Patent Literature 6: WO2021/117839

Patent Literature 7: WO2021/070875

SUMMARY OF INVENTION

Technical Problem

However, the present inventors have found that the compositions according to Patent Literatures 4 to 7 need further improvement to achieve both cavity sealing properties and shade conformity when applied to cavities in wedge-shaped defects.

Wedge-shaped defects are a form of tooth wear, usually non-carious. When wedge-shaped defects occur, it often exposes the dentin, causing pain during brushing or leading to sensitivity as the condition worsens.

The causes of wedge-shaped defects include (1) wear of the very thin enamel around the cervical region from using an abrasive dentifrice with a hard toothbrush, (2) wear of the enamel, dentin, and cementum near the cervical region due to brushing such as vigorous horizontal brushing, and (3) occlusal forces (abfraction), such as those from bruxism, where tensile stress concentrates on the cervical area due to occlusal forces, leading to the breakdown of tooth structure and defective tooth structure in the cervical region.

One characteristic of wedge-shaped defect cavities is that the edge (margin) includes both enamel and dentin within a single cavity. In common cavities requiring filling, the margin is either enamel for crown caries or dentin for root caries, allowing for easy color matching using a single shade. However, cavities in wedge-shaped defects require shade conformity to both enamel and dentin, which significantly differ in shade, within a single cavity.

Generally, the ability to seal cavities (hereinafter, also referred to as "cavity sealing properties") is determined by the adhesion to tooth structure and the extent of polymerization shrinkage. In ordinary cavities, if adhesion to the enamel is weak, the composite resin filling the cavity detaches itself from the margin, whereas the composite resin separates from the base of the cavity if adhesion to the dentin is weak, leading to symmetrical detachment in all directions.

However, in wedge-shaped defect cavities, the composite resin filling the cavity separates from the crown margin if adhesion to the enamel is weak, whereas the composite resin detaches itself from the root margin if it has weak adhesion to the dentin. This results in detachment beginning from either the top or bottom. In such situations, the presence of moisture or other stressing factors cause detachment to progress on one side, increasing the risk of detachment. That is, cavities in wedge-shaped defects can be said as having a higher risk of detachment compared to ordinary cavities.

Considering the high risk of detachment, the restorative treatment of cavities in wedge-shaped defects thus requires cavity sealing properties exceeding the levels typically demanded for ordinary cavities, in order to allow for the broader application of self-adhesive dental composite resin without limiting its use to the restorative treatment of enamel or dentin cavities.

As described above, the restorative treatment of wedge-shaped defect cavities involves specific challenges in terms of both shade conformity and cavity sealing properties.

It is an object of the present invention to provide a self-adhesive dental composite resin that excels in bond strength to tooth structure, cavity sealing properties, and shade conformity.

Another object of the present invention is to provide a self-adhesive dental composite resin that can achieve both favorable cavity sealing properties and good shade conformity when used for cavities in wedge-shaped defect.

Solution to Problem

The present inventors conducted intensive studies, and found that the foregoing issues can be solved with a self-adhesive dental composite resin satisfying specific parameters. This led to the completion of the present invention after further examinations.

The present invention includes the following.

[1] A self-adhesive dental composite resin comprising a monomer (a) having an acidic group, a monomer (b) having no acidic group, a polymerization initiator (c), and a filler (d),

wherein the self-adhesive dental composite resin has a polymerization shrinkage stress of less than 10.0 MPa, and

the degree of light diffusion D as indicated by the following formula [I] for the self-adhesive dental composite resin after curing is 0.10 or more and less than 3.00,

$$D = (I_{20}/\cos 20° + I_{70}/\cos 70°)/(2I_0) \qquad [I]$$

where I represents the luminous intensity of light transmitted through a sample plate made of the self-adhesive dental composite resin after curing, and $I_0$, $I_{20}$, and $I_{70}$ represent the luminous intensity values (intensity of light) at 0-degree, 20-degree, and 70-degree angles, respectively, relative to the direction perpendicular to the sample plate (the direction of light incidence).

[2] The self-adhesive dental composite resin according to [1], which has a water absorption of less than 40 $\mu g/mm^3$ in its cured product based on ISO4049:2019.
[3] The self-adhesive dental composite resin according to [1] or [2], which has a shear bond strength of 4.0 MPa or more to dentin based on ISO29022:2013.
[4] The self-adhesive dental composite resin according to any one of [1] to [3], which has a shear bond strength of 4.0 MPa or more to enamel based on ISO29022:2013.
[5] The self-adhesive dental composite resin according to any one of [1] to [4], which has a flexural strength of 80 MPa or more in its cured product based on ISO4049:2019.
[6] The self-adhesive dental composite resin according to any one of [1] to [5], wherein the monomer (b) having no acidic group comprises a monofunctional hydrophobic monomer (b-1a).
[7] The self-adhesive dental composite resin according to any one of [1] to [6], wherein the filler (d) comprises two or more fillers with different types and/or proportions of constituents.
[8] The self-adhesive dental composite resin according to any one of [1] to [7], wherein the filler (d) comprises two or more fillers with an average particle diameter of 0.1 $\mu$m or more and different refractive indices.
[9] The self-adhesive dental composite resin according to [8], wherein the fillers (d) with different refractive indices have a refractive index difference of 0.02 or more, and the refractive indices of the fillers (d) with different refractive indices are 1.40 or more and 1.70 or less.
[10] The self-adhesive dental composite resin according to [8] or [9], wherein one or more of the fillers with different refractive indices comprise at least one metallic element selected from the group consisting of aluminum, strontium, zirconium, barium, lanthanum, ytterbium, titanium, and bismuth.
[11] The self-adhesive dental composite resin according to any one of [1] to [10], wherein the filler (d) comprises a filler (d-i) with an average particle diameter of 1 nm or more and less than 0.1 $\mu$m.
[12] The self-adhesive dental composite resin according to any one of [1] to [11], wherein the monomer (a) having an acidic group comprises a monomer having a phosphoric acid group.
[13] The self-adhesive dental composite resin according to any one of [1] to [12], wherein the filler (d) comprises silica-coated ytterbium fluoride.
[14] The self-adhesive dental composite resin according to any one of [1] to [13], wherein the polymerization initiator (c) comprises a water-soluble photopolymerization initiator.
[15] The self-adhesive dental composite resin according to any one of [1] to [14], wherein the monomer (b) having no acidic group comprises a (meth)acrylic compound (b-3) having a weight-average molecular weight of 1,000 or more and less than 80,000, and a weight-average molecular weight of 1,250 or more and less than 20,000 per polymerizable group.

Advantageous Effects of Invention

The self-adhesive dental composite resin excels in bond strength to tooth structure, and exhibits favorable cavity sealing properties, making it applicable as a dental restoration material for cavities in the treatment of ordinary dental caries. Furthermore, a self-adhesive dental composite resin of the present invention possesses light diffusing properties, allowing reflected and diffused light to be seen as manifesting the shade of the dental composite material or its background color. This produces superior blending effects, softening the restoration's background color or the boundary between the restoration and natural teeth, thereby demonstrating excellent shade conformity to the surrounding tooth structure of cavities in dental treatment (for example, the treatment of dental caries). That is, a self-adhesive dental composite resin of the present invention possesses high adaptability with no limitation to the location of the cavity, making it applicable to cavities in enamel and in dentin, and those spanning enamel and dentin.
A self-adhesive dental composite resin of the present invention also exhibits excellent durability in cavity sealing properties.
Additionally, a self-adhesive dental composite resin of the present invention shows low water absorption and excellent durability in mechanical strength (for example, flexural strength).
According to the present invention, a self-adhesive dental composite resin can be provided that can achieve both favorable cavity sealing properties and good shade conformity in wedge-shaped defect cavities.

## BRIEF DESCRIPTION OF DRAWINGS

[FIG. 1] An explanatory diagram in relation to a method of measurement of degree of light diffusion D according to the present invention.

[FIG. 2] A schematic view illustrating how a glass transition temperature is determined when glass transition shows a step-like change as in FIG. 3 of JIS K 7121-1987 representing a method of determination of glass transition temperature.

## DESCRIPTION OF EMBODIMENTS

A self-adhesive dental composite resin of the present invention comprises a monomer (a) having an acidic group, a monomer (b) having no acidic group, a polymerization initiator (c), and a filler (d),

wherein the self-adhesive dental composite resin has a polymerization shrinkage stress of less than 10.0 MPa, and the degree of light diffusion D for the self-adhesive dental composite resin after curing is 0.10 or more and less than 3.00.

The term "(meth)acryl" as used in the present specification collectively refers to methacryl and acryl. The same applies to similar expressions (such as (meth)acrylic acid, and (meth)acrylonitrile). In this specification, the upper limits and lower limits of numeric ranges (for example, ranges of contents of components, ranges of values calculated from components, and ranges of physical properties) can be appropriately combined. In this specification, various modifications can be made to the embodiments, such as by appropriately combining all or part of the embodiments.

In this specification, lightness and chromaticity refer to lightness (L*) and chromaticity (a*, b*), respectively, according to CIE LAB (JIS Z 8781-4:2013 Color Measurements - Part 4: CIE 1976 L*a*b* color space).

It remains somewhat unclear why a self-adhesive dental composite resin of the present invention can exhibit excellent bond strength to tooth structure and superior cavity sealing properties and shade conformity, and achieve notable cavity sealing properties and shade conformity when used for cavities in wedge-shaped defects. However, the following speculation has been made.

When self-adhesive dental composite resin is filled into cavities, the monomer containing an acidic group binds to the tooth structure, leading to adhesion to the tooth structure through polymerization of the self-adhesive dental composite resin. During polymerization, the self-adhesive dental composite resin undergoes shrinkage, creating shrinkage stress in the direction opposite its adhesion to the tooth structure. In order to demonstrate outstanding cavity sealing properties, it is considered necessary for the bond strength to surpass the polymerization shrinkage stress. It is believed that using a self-adhesive dental composite resin with low polymerization shrinkage stress, as in the present invention, reduces the effects of polymerization shrinkage stress, thereby achieving excellent cavity sealing properties in the restorative treatment of wedge-shaped defect cavities.

When using self-adhesive dental composite resin on tooth structure, it is aesthetically desirable for the boundary between the tooth structure and self-adhesive dental composite resin to be indistinguishable. A self-adhesive dental composite resin of the present invention exhibits adequate light diffusing properties after curing. This causes some of the light to diffuse on the self-adhesive composite resin, obscuring the boundary with the tooth structure. This probably explains the favorable shade conformity to the surrounding tooth structure of cavities in dental treatment (for example, the treatment of dental caries), particularly between enamel and dentin, which significantly differ in shade.

A self-adhesive dental composite resin of the present invention must have a polymerization shrinkage stress lower than a predetermined value. Polymerization shrinkage stress refers to the force exerted when radical polymerizable curable compositions such as self-adhesive dental composite resins undergo shrinkage upon curing. The cavity sealing properties depend on bond strength to tooth structure, and the extent of polymerization shrinkage stress. Smaller values of polymerization shrinkage stress are preferred.

Polymerization shrinkage stress also depends on the type, mixing ratio, and viscosity of monomers, the type and amount of polymerization initiators, the type and amount of polymerization accelerators, the ratio of polymerization initiators and polymerization accelerators, and the type, particle size, and content of fillers. Furthermore, polymerization shrinkage stress also varies based on factors such as the type and content of additives (ultraviolet absorbers, polymerization inhibitors, pigments, fluorescent agents), the paste properties and transparency of self-adhesive dental composite resin, and the quantity of light from dental visible-light irradiators. These factors interact in a complex manner to affect polymerization shrinkage stress. In particular, the type of monomers, and the amount of polymerization initiators and/or polymerization accelerators have a significant impact among these factors.

Below is a description regarding the adjustment of polymerization shrinkage stress.

Generally, monomers with a larger number of polymerizable groups and more rigid skeletons (for example, aromatic rings) lead to increased polymerization shrinkage stress. The polymerization shrinkage stress also increases as the viscosity decreases. Furthermore, these are influenced by the proportion of monomer components. The type of

monomer, and its mixing ratio and viscosity can be appropriately adjusted taking into consideration the desired balance of physical properties, with consideration given to the flexural strength and other properties of the cured product.

Generally, the polymerization shrinkage stress increases with increasing amounts of polymerization initiators. Moreover, it increases when the wavelength range of dental visible-light irradiators more closely matches the absorption wavelength of polymerization initiators. Higher quantities of polymerization accelerators also lead to greater polymerization shrinkage stress, and, depending on its proportion relative to the polymerization initiator, the polymerization shrinkage stress increases as a result of improved polymerization rates.

When the filler is, for example, a surface-treated filler (for example, those containing silica as a main component), the presence of polymerizable groups in surface treatment agents leads to a larger number of polymerizable group on the treated filler surface, potentially increasing the polymerization shrinkage stress. However, the polymerization shrinkage stress can be reduced by adjusting parameters such the type of monomer components and filler content. That is, the type of filler and surface treatment agent can be appropriately changed taking into consideration the desired balance of physical properties such as the flexural strength of the cured product.

Polymerization shrinkage stress increases with an increase in the hardness of the filler. As the filler content increases, the cured product becomes harder, and the polymerization shrinkage stress increases. Conversely, reducing the filler content increases the amount of monomer, potentially leading to greater polymerization shrinkage, and, consequently, higher polymerization shrinkage stress.

A larger filler particle size tends to make the cured product harder and increase polymerization shrinkage stress. Conversely, a smaller filler particle size increases the specific surface area of particles, reducing the filler content and increasing monomer content. This may also lead to an increase of polymerization shrinkage stress as above.

Increasing the content of ultraviolet absorbers and polymerization inhibitors slows down the rate of polymerization, resulting in lower polymerization shrinkage stress. Similarly, higher contents of pigments and fluorescent agents lead to a decrease in light transmissivity. This tends to decrease the polymerization rate, leading to lower polymerization shrinkage stress. The type and content of additives can be appropriately varied taking into consideration the desired balance of physical properties, including the aesthetics of the cured product.

When the self-adhesive dental composite resin exhibits high transparency before curing, it tends to allow light to pass through easily during curing, leading to increased polymerization shrinkage stress.

Additionally, lower viscosity in the paste encourages shrinkage during polymerization, and tends to increase polymerization shrinkage stress.

In view of cavity sealing properties, the polymerization shrinkage stress is less than 10.0 MPa, preferably less than 9.5 MPa, more preferably less than 9.0 MPa, even more preferably less than 8.5 MPa. The method of measurement of polymerization shrinkage stress is as described in the EXAMPLES section below.

A self-adhesive dental composite resin of the present invention must possess predetermined light diffusing properties in a cured state. The light diffusing properties refer to the ability to diffuse light in various directions by allowing light to be refracted and reflected by the filler materials within semi-transparent materials such as dental composite materials when light is incident on such materials. This allows reflected and diffused light to be seen as manifesting the shade of the dental composite material or its background color. It is believed that higher light diffusing properties lead to enhanced blending effect, more effectively softening the restoration's background color or the boundary between the restoration and natural teeth, thereby demonstrating superior shade conformity to both enamel and dentin. As an index of the light diffusing properties, a degree of light diffusion D defined by the following formula [I] has been proposed. FIG. 1 is an explanatory diagram illustrating formula [I].

$$D = (I_{20}/\cos 20° + I_{70}/\cos 70°)/(2I_0) \qquad [I]$$

where I represents the luminous intensity of light transmitted through a sample plate made of the self-adhesive dental composite resin after curing, and $I_0$, $I_{20}$, and $I_{70}$ represent the luminous intensity values (intensity of light) at 0-degree, 20-degree, and 70-degree angles, respectively, relative to the direction perpendicular to the sample plate (the direction of light incidence).

The luminous intensity (intensity of light) can be measured using a goniometer or goniophotometer. A higher value of degree of light diffusion D indicates higher light diffusing properties in the cured product. The goniometer may be, for example, a goniometer GP-200 manufactured by Murakami Color Research Laboratory Co., Ltd.

A self-adhesive dental composite resin of the present invention has a degree of light diffusion D of 0.10 or more, preferably 0.12 or more, more preferably 0.15 or more, even more preferably 0.18 or more, particularly preferably 0.20 or more in its cured product. The cured product has a degree of light diffusion D of less than 3.00, preferably 2.80 or less, more preferably 2.50 or less, even more preferably 2.00 or less. In view of even superior shade conformity to both enamel and dentin, the degree of light diffusion D is particularly preferably 1.00 or less. When the degree of light diffusion D is less than 0.10, the self-adhesive dental composite resin exhibits insufficient light diffusing properties, resulting in inadequate shade

conformity to enamel and dentin. When the degree of light diffusion D is more than 3.00, it may lead to overly strong light diffusing properties, resulting in insufficient transparency. The measurement method for the degree of light diffusion D of the cured product is as described in the EXAMPLES section below.

In a self-adhesive dental composite resin of the present invention, the degree of light diffusion D can be confined within the foregoing ranges by adjusting the refractive index difference between fillers (d), and the refractive index difference between the monomer components and fillers (d). As a rule, when there is a smaller difference in refractive index, the degree of light diffusion D tends to decrease. However, the degree of light diffusion D is also influenced by factors other than refractive index difference, including the type or content of monomer components, and the particle size or shape of filler (d). For example, fillers with larger particle sizes are more likely to increase the degree of light diffusion D by facilitating easier diffusion of light of visible light wavelengths. The degree of light diffusion D also tends to increase with crushed fillers because these fillers diffuse light in multiple directions. Easier adjustment of degree of light diffusion D is possible by reducing the content of monomer components and increasing the content of filler (d). The degree of light diffusion D is particularly susceptible to the refractive index difference between fillers (d).

Preferably, a self-adhesive dental composite resin of the present invention is low in water absorption. Water absorption refers to the amount of water absorbed by cured products of radical polymerizable curable compositions such as self-adhesive dental composite resins. The durability of mechanical strength (for example, flexural strength) is influenced by water absorption. Higher water absorption leads to more water being contained in the cured product, making it softer and more brittle. On the other hand, lower water absorption means that the cured product contains less water, which helps maintain mechanical strength. In this respect, it is preferable to have lower water absorption.

Water absorption also depends on the type, mixing ratio, and viscosity of monomers, the type and amount of polymerization initiators, the type and amount of polymerization accelerators, the ratio of polymerization initiators and polymerization accelerators, and the type, particle size, and content of fillers. In particular, the type of monomers, and the type, particle size, and content of fillers have a significant impact among these factors, aside from surface treatment.

Below is a description regarding the adjustment of water absorption.

Generally, monomers with a larger number of hydrophilic functional groups such as hydroxyl groups, ether bonds, and amines lead to increased water absorption. Monomers with no such functional groups lead to low water absorption. A high proportion of monomers with hydrophilic functional groups may lead to increased polymerization shrinkage stress. For example, using monomers with hydrophilic functional groups may lead to higher polymerization shrinkage stress due to the high curability attributed to these hydrophilic functional groups. This means that water absorption can decrease when the amount of monomers with hydrophilic functional groups is reduced. Together with other components, this can lead to a decrease in polymerization shrinkage stress.

As the particle size of the filler increases, the specific surface area decreases, leading to less water adhering to the surface and thereby reducing water absorption. Conversely, smaller particles sizes result in more water adhering to the surface, increasing water absorption. Surface treatment makes the filler surface more hydrophobic, and decreases water absorption. Water absorption decreases further as the surface becomes more hydrophobic. One possible way of increasing the hydrophobicity of filler is to promote dehydrocondensation reaction through surface treatment with the use of fillers with a large number of surface hydroxyl groups (for example, those containing silica as a main component). Alternatively, water absorption can be reduced with the use of fillers with high hydrophobicity.

In terms of increasing water absorption, hydrophilic monomers have a more significant impact than fillers. As a result, higher filler content and lower hydrophilic monomer content leads to lower water absorption, whereas lower filler content and higher hydrophilic monomer content tends to increase water absorption. Generally, organic fillers tend to lead to higher water absorption compared to inorganic fillers and organic-inorganic composite fillers. In terms of achieving a greater reduction of water absorption, a certain embodiment is, for example, a self-adhesive dental composite resin that does not comprise an organic filler.

In view of the durability of the cured product, lower water absorption is preferable. The water absorption of the cured product is preferably less than 40 $\mu g/mm^3$, more preferably 30 $\mu g/mm^3$ or less, even more preferably 20 $\mu g/mm^3$ or less, particularly preferably 15 $\mu g/mm^3$ or less. The water absorption of the cured product can be measured according to ISO4049:2019. The method of measurement of water absorption is as described in the EXAMPLES section below.

It is preferable for a self-adhesive dental composite resin of the present invention to exhibit high bond strength to tooth structure. Bond strength to tooth structure refers to the shear bond strength between the adhesive material and tooth structure, and it influences the cavity sealing properties. A higher bond strength ensures stronger bonding to the tooth structure, preventing detachment due to polymerization shrinkage stress during curing. This results in superior cavity sealing properties, in addition to increasing the durability of cavity sealing properties. Conversely, a weaker bond strength may lead to detachment of the adhesive material from tooth structure due to factors such as polymerization shrinkage stress and thermal load, reducing the cavity sealing properties. It is therefore preferable to increase the bond strength to tooth structure.

The bond strength to tooth structure also depends on the type, mixing ratio, and viscosity of monomers, the type and amount of polymerization initiators, the type and amount of polymerization accelerators, the ratio of polymerization

initiators and polymerization accelerators, and the type, amount, particle size, and content of fillers. In particular, the type of monomers, the type of polymerization initiators, and the type, particle size, and content of fillers have a significant impact among these factors.

Below is a description regarding the adjustment of bond strength to tooth structure.

Generally, monomers with hydrophilic functional groups such as hydroxyl groups, ether bonds, and amines exhibit higher wettability to tooth structure, leading to increased bond strength. Likewise, polymerization initiators with higher hydrophilicity tend to increase bond strength. These two factors affect bond strength to both enamel and dentin, with a more pronounced effect on bond strength to dentin in particular.

The metals contained in certain types of fillers can form salts with the acidic groups present in the monomer (a) having an acidic group, reducing the number of acidic groups and potentially lowering the bond strength to tooth structure. Considering this, fillers that are less prone to reducing the bond strength to tooth structure (for example, those containing silica as a main component) can be selected or combined, taking into account the balance with other physical properties.

The bond strength to tooth structure is also influenced by the flowability of self-adhesive dental composite resin. Higher viscosity in self-adhesive dental composite resin tends to reduce the flowability of the components within the paste, leading to decreased bond strength to tooth structure. Lower viscosity in monomer components tends to increase flowability. The flowability tends to increase with lower filler content, and the specific surface area decreases with larger particle size. These tend to lead to increased flowability of the components within the paste.

In view of cavity sealing properties and the durability of cavity sealing properties in wedge-shaped defect cavities, it is preferable to have higher shear bond strength to enamel and dentin. The shear bond strength to enamel and dentin is preferably 4.0 MPa or more, more preferably 5.0 MPa or more, even more preferably 6.0 MPa or more, particularly preferably 8.0 MPa or more. The shear bond strength to enamel and dentin can be measured according to ISO29022:2013. The method of measurement of bond strength to tooth structure (enamel and dentin) is as described in the EXAMPLES section below.

It is preferable for a self-adhesive dental composite resin of the present invention to exhibit high flexural strength in its cured product. Flexural strength refers to the strength measured for the cured product of the self-adhesive dental composite resin in a three-point flexural test, and contributes to the mechanical strength of the self-adhesive dental composite resin. The self-adhesive dental composite resin exhibits high strength when it has high flexural strength, enabling its cured product to withstand loads such as occlusion without breaking. Low flexural strength may lead to fracture in the cured product.

The flexural strength also depends on the type and mixing ratio of monomers, the type and amount of polymerization initiators, the type and amount of polymerization accelerators, the ratio of polymerization initiators and polymerization accelerators, and the type, amount, particle size, and content of fillers. In particular, the type of monomers, and the type, particle size, and content of fillers have a significant impact among these factors.

Below is a description regarding the adjustment of flexural strength.

Generally, monomers with a larger number of polymerizable groups lead to increased flexural strength. The flexural strength also increases when monomers contain rigid skeletons (for example, aromatic rings) and possess hydrogen bonding capabilities. These are also influenced by the proportion of monomer components. Generally, the flexural strength increases with increasing amounts of polymerization initiators.

Higher quantities of polymerization accelerators also lead to greater flexural strength, and, depending on its proportion relative to the polymerization initiator, the flexural strength further increases as a result of improved polymerization conversion rate. The content of polymerization accelerator can be appropriately adjusted, taking into consideration a balance between polymerization shrinkage stress and flexural strength.

The flexural strength tends to increase when the filler is surface-treated. To increase flexural strength, fillers that are suited for surface treatment (for example, those containing silica as a main component) can be selected or combined. On the other hand, increasing the number of polymerizable groups on filler surface to increase flexural strength leads to increased polymerization shrinkage stress. The type of filler and the type of surface treatment can be appropriately varied, taking into account a balance between polymerization shrinkage stress and flexural strength.

Fillers with higher hardness lead to greater flexural strength, and increasing the filler content increases flexural strength by making the cured product harder. Fillers with larger particle sizes can lead to higher flexural strength because such fillers tend to increase the hardness of the cured product. Higher contents of polymerization inhibitors reduce polymerization conversion rates, resulting in lower flexural strength.

In view of the mechanical strength of the cured product, the flexural strength is preferably 80 MPa or more, more preferably 85 MPa or more, even more preferably 90 MPa or more, particularly preferably 95 MPa or more, most preferably 100 MPa or more. The flexural strength of the cured product can be measured according to ISO4049:2019. The method of measurement of flexural strength is as described in the EXAMPLES section below.

The following describes the components used in a self-adhesive dental composite resin of the present invention.

<Monomer (a) Having Acidic Group>

In view of adhesive properties to tooth structure and cavity sealing properties, a monomer (a) having an acidic group is essential in a self-adhesive dental composite resin of the present invention. By incorporating a monomer (a) having an acidic group, it is possible to achieve superior cavity sealing properties and excellent durability of cavity sealing properties, with particularly superior sealing properties for cavities in wedge-shaped defects. Preferred for use in the self-adhesive dental composite resin are radical polymerizable monomers. Specific examples of radical polymerizable monomers in monomer (a) having an acidic group include (meth)acrylate monomers, (meth)acrylamide monomers, esters, vinyl esters, and vinyl ethers of acids such as $\alpha$-cyanoacrylic acid, (meth)acrylic acid, $\alpha$-halogenated acrylic acid, crotonic acid, cinnamic acid, sorbic acid, maleic acid, and itaconic acid, and mono-N-vinyl derivatives and styrene derivatives. In view of curability, preferred are (meth)acrylate monomers and (meth)acrylamide monomers.

Examples of the monomer (a) having an acidic group used in the present invention include monomers having at least one acidic group such as a phosphoric acid group, a pyrophosphoric acid group, a thiophosphoric acid group, a phosphonic acid group, a carboxylic acid group, and a sulfonic acid group. The monomer (a) having an acidic group may be used alone, or two or more thereof may be used in appropriate combinations. Specific examples of the monomer (a) having an acidic group are as follows.

Examples of monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl-2-dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, 2-methacryloyloxypropyl-(4-methoxyphenyl)hydrogen phosphate, and acid chlorides, alkali metal salts, and amine salts of these. Preferred are monomers having divalent phosphoric acid groups with C6 to C12 alkylene groups. A certain preferred embodiment is, for example, a self-adhesive dental composite resin in which the monomer (a) having an acidic group comprises a monomer having a divalent phosphoric acid group with a C6 to C12 alkylene group.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of monomers having a carboxylic acid group include monofunctional (meth)acrylic acid esters having one carboxyl group or acid anhydride group thereof per molecule, and monofunctional (meth)acrylic acid esters having a plurality of carboxyl groups or acid anhydride groups thereof per molecule.

Examples of monofunctional monomers having one carboxyl group or acid anhydride group thereof per molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, and N-(meth)acryloyl-4-aminosalicylic acid, and compounds derived by converting the carboxyl group of the aforementioned compounds into an acid anhydride group.

Examples of monofunctional monomers having a plurality of carboxyl groups or acid anhydride groups thereof

per molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth) acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propylsuccin ate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-naphthalic acid anhydride, and 4-(meth)acryloyloxyethylnaphthalene-1,8-tricarboxylic acid anhydride.

Examples of monomers having a sulfonic acid group include 2-sulfoethyl (meth)acrylate.

In view of providing good bond strength when the monomer (a) having an acidic group is used as a component of the self-adhesive dental composite resin, preferred among the foregoing monomers (a) having an acidic group are monomers having a phosphoric acid group, or monomers having a carboxylic acid group, more preferably 2-(meth) acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyltrimellitate anhydride, 4-(meth)acryloyloxyethyltrimellitate, 11-(meth)acryloyloxyundecane-1,1-dicarboxylic acid, and a mixture of 2-methacryloyloxyethyl dihydrogen phosphate and bis(2-methacryloyloxyethyl)hydrogen phosphate, even more preferably 8-(meth) acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is particularly preferred.

In view of adhesive properties to tooth structure and cavity sealing properties (particularly, cavity sealing properties in wedge-shaped defect cavities), as well as durability of cavity sealing properties, the content of the monomer (a) having an acidic group in the self-adhesive dental composite resin is preferably 1 to 40 parts by mass, more preferably 2 to 35 parts by mass, even more preferably 3 to 30 parts by mass, particularly preferably 5 to 25 parts by mass in total 100 parts by mass of monomers in the self-adhesive dental composite resin.

<Monomer (b) Having no Acidic Group>

Examples of the monomer (b) having no acidic group in the present invention include hydrophobic monomers (b-1) having no acidic group and having a solubility of less than 10 mass% in 25°C water (hereinafter, also referred to simply as "hydrophobic monomers (b-1)"), and hydrophilic monomers (b-2) having no acidic group and having a solubility of 10 mass% or more in 25°C water (hereinafter, also referred to simply as "hydrophilic monomers (b-2)"). The monomer (b) having no acidic group may be used alone, or two or more thereof may be used in combination.

· Hydrophobic Monomer (b-1) Having no Acidic Group

The hydrophobic monomer (b-1) having no acidic group improves properties such as handling of the self-adhesive dental composite resin, and the mechanical strength (flexural strength) of the cured product, and reduces water absorption and dissolution. The hydrophobic monomer (b-1) is preferably a radical polymerizable monomer having no acidic group but having a polymerizable group. In view of ease of radical polymerization, preferred as the polymerizable group are (meth)acryloyloxy groups and/or (meth)acrylamide groups. The hydrophobic monomer (b-1) means a monomer that has no acidic group, and has a solubility of less than 10 mass% in 25°C water. The hydrophobic monomer can be classified into monofunctional hydrophobic monomer (b-1a) and polyfunctional hydrophobic monomer (b-1b). Examples of the monofunctional hydrophobic monomer (b-1a) include aromatic monofunctional monomers, and aliphatic monofunctional monomers. Examples of the polyfunctional hydrophobic monomer (b-1b) include crosslinkable monomers such as aromatic bifunctional monomers, aliphatic bifunctional monomers, and tri- and higher-functional monomers. The hydrophobic monomer (b-1) may be used alone, or two or more thereof may be used in combination.

· Monofunctional Hydrophobic Monomer (b-1a)

The monofunctional hydrophobic monomer (b-1a) is preferred in terms adjusting the viscosity and refractive index of the composition, and reducing polymerization shrinkage stress, water absorption, and dissolution. Examples of the monofunctional hydrophobic monomer (b-1a) include (meth)acrylate monomers, and (meth)acrylamide monomers. The monofunctional hydrophobic monomer (b-1a) may be used alone, or two or more thereof may be used in combination.

Examples of the monofunctional hydrophobic monomer (b-1a) include:

aliphatic monofunctional (meth)acrylate monomers such as n-stearyl methacrylate;
aliphatic monofunctional (meth)acrylate monomers containing an ether bond(s), such as butoxydiethylene glycol methacrylate, and methoxypolyethylene glycol methacrylate (with an average of nine moles of oxyethylene group added);
alicyclic monofunctional (meth)acrylate monomers such as cyclohexyl methacrylate, isobornyl methacrylate, and dicyclopentanyl methacrylate;
monofunctional (meth)acrylate monomers having an aromatic ring group(s), such as benzyl (meth)acrylate, 2-phenoxyethyl (meth)acrylate, and phenoxybenzyl (meth)acrylate; and
(meth)acrylate monomers containing a heterocyclic ring group(s) (for example, cyclic ether groups), such as tetrahydrofurfuryl methacrylate.

The monofunctional (meth)acrylate monomers having an aromatic ring group(s) are preferably those containing one or two phenyl groups. The (meth)acrylate monomers containing a heterocyclic ring group(s) are preferably those containing one or two heterocyclic ring groups (for example, cyclic ether groups). In view of the effect to reduce mechanical strength and polymerization shrinkage stress, preferred among these are tetrahydrofurfuryl methacrylate (commonly known as THF-MA), benzyl methacrylate (commonly known as BEMA), phenoxybenzyl methacrylate (commonly known as POB-MA), and 2-phenoxyethyl methacrylate (commonly known as PEMA).

· Polyfunctional Hydrophobic Monomer (b-1b)

The polyfunctional hydrophobic monomer (b-1b) is used to improve the mechanical strength and handling properties of the cured product of the composition, and adjust the refractive index of the monomer components. Examples of the polyfunctional hydrophobic monomer (b-1b) include aromatic bifunctional hydrophobic monomers, aliphatic bifunctional hydrophobic monomers, and tri- and higher-functional hydrophobic monomers.
The polyfunctional hydrophobic monomer (b-1b) may be used alone, or two or more thereof may be used in combination.
Examples of the aromatic bifunctional hydrophobic monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.
In view of adjusting mechanical strength and refractive index and from the perspective of handling properties, preferred among these are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.
Examples of the aliphatic bifunctional hydrophobic monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)di(meth)acrylate, N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.
In view of mechanical strength and handling properties, preferred are triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), and 1,10-decanediol dimethacrylate (commonly known as DD). Preferred in view of polymerization shrinkage stress are 1,2-bis(3-methacryloyloxy-2-hydroxypropoxy)ethane, UDMA, and DD. MAEA, and N-methacryloyloxypropylacrylamide are preferred in view of adhesive properties to tooth structure, particularly dentin.
Examples of the tri- and higher-functional hydrophobic monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acr ylate, and 1,7-diacryloyloxy-2,2,6,6-tetra(meth)acryloyloxymethyl-4-oxaheptane. In view of mechanical strength, N,N-(2,2,4-trimethylhexamethylene)bis[2-(aminocarboxy)propane-1,3-diol]tetramethacryl ate is preferred.

In view of the mechanical strength (flexural strength), water absorption, dissolution, and handling properties, preferred for use among these hydrophobic monomers (b-1) are aromatic bifunctional hydrophobic monomers, aliphatic bifunctional hydrophobic monomers, and monofunctional hydrophobic monomers. Preferred as aromatic bifunctional monomers are Bis-GMA and D-2.6E. Preferred as aliphatic bifunctional monomers are 3G, neopentyl glycol di(meth)acrylate, UDMA, DD, and MAEA. Preferred as monofunctional hydrophobic monomers are THF-MA, BEMA, POB-MA, and PEMA.

In view of cavity sealing properties (particularly, cavity sealing properties in wedge-shaped defect cavities) and its durability when combined with other components, and the effect to reduce water absorption and polymerization shrinkage stress in applications as self-adhesive dental composite resins, preferred among the foregoing hydrophobic monomers (b-1) are hydrophobic monomers with no hydroxyl group (monofunctional hydrophobic monomers with no hydroxyl group, aromatic bifunctional hydrophobic monomers with no hydroxyl group, and aliphatic bifunctional hydrophobic monomers with no hydroxyl group), more preferably D-2.6E, DD, UDMA, MAEA, THF-MA, BEMA, POB-MA, and PEMA, even more preferably D-2.6E, DD, MAEA, and THF-MA.

The hydrophobic monomer (b-1) may be incorporated alone, or two or more thereof may be used in combination. When the content of hydrophobic monomer (b-1) is at or below the foregoing upper limits, a decrease of adhesion due to reduced wettability of the self-adhesive dental composite resin to tooth structure can more easily be reduced, whereas the cured product can more easily exhibit the desired mechanical strength and handling properties when the content of hydrophobic monomer (b-1) is at or above the foregoing lower limits. The content of the hydrophobic monomer (b-1) in the self-adhesive dental composite resin is preferably 20 to 98 parts by mass, more preferably 40 to 95 parts by mass, even more preferably 60 to 92 parts by mass in total 100 parts by mass of monomers in the self-adhesive dental composite resin.

· Hydrophilic Monomer (b-2) Having no Acidic Group

The hydrophilic monomer (b-2) can enhance the bond strength to tooth structure by improving the wettability of the self-adhesive dental composite resin to tooth structure, and the penetration of the self-adhesive dental composite resin into tooth structure (enamel/dentin). The hydrophilic monomer (b-2) is preferably a radical polymerizable monomer having no acidic group but having a polymerizable group. In view of ease of radical polymerization, the polymerizable group is preferably a (meth)acryloyloxy group and/or a (meth)acrylamide group. The hydrophilic monomer (b-2) means a monomer that has no acidic group, and that has a solubility of 10 mass% or more in 25°C water. The hydrophilic monomer (b-2) is preferably one having a solubility of 30 mass% or more in 25°C water, more preferably one that can dissolve in water in any proportions at 25°C. The hydrophilic monomer is preferably one having a hydrophilic group such as a hydroxyl group, an oxymethylene group, an oxyethylene group, an oxypropylene group, or an amide group.

Examples of the hydrophilic monomer (b-2) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with an average of nine or more moles of oxyethylene group added); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (b-2) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide. The hydrophilic monomer (b-2) may be incorporated alone, or two or more thereof may be used in combination.

The content of the hydrophilic monomer (b-2) in the self-adhesive dental composite resin is preferably 0 to 50 parts by mass, more preferably 0 to 40 parts by mass, even more preferably 0 to 30 parts by mass in total 100 parts by mass of the monomers. The content of hydrophilic monomer (b-2) may be 0 parts by mass in total 100 parts by mass of the monomers. When the content of the hydrophilic monomer (b-2) in the self-adhesive dental composite resin is at or above these lower limits, it is easier to produce an effect that sufficiently improves the bond strength, whereas the cured product can more easily exhibit the desired mechanical strength, water absorption, and dissolution when the content of hydrophilic monomer (b-2) is at or below the foregoing upper limits.

[(Meth)Acrylic Compound (b-3) Having Weight-Average Molecular Weight of 1,000 to 80,000, and Weight-Average Molecular Weight of 1,250 or More and Less Than 20,000 per Polymerizable Group]

In a self-adhesive dental composite resin of the present invention, the (meth)acrylic compound (b-3) having a weight-average molecular weight of 1,000 to 80,000, and a weight-average molecular weight of 1,250 or more and less than 20,000 per polymerizable group (hereinafter, referred to as (meth)acrylic compound (b-3)) is used to impart low polymerization shrinkage stress, and superior cavity sealing properties (particularly, cavity sealing properties in wedge-shaped defect cavities), as well as durability of cavity sealing properties. In the context of monomer (b) having no acidic group, (meth)acrylic compounds with a weight-average molecular weight of 1,000 to 80,000, and a weight-average molecular weight of 1,250 or more and less than 20,000 per polymerizable group are classified as (meth)acrylic compounds (b-3) in this specification, irrespective of the solubility in 25°C water.

The (meth)acrylic compound (b-3) can be broadly classified into urethanized (meth)acrylic compound (b-3a) and (meth)acrylic compound (b-3b) having no urethane skeleton. The urethanized (meth)acrylic compound (b-3a) is preferred in view of ease of introducing of a (meth)acryl group, and the effect to reduce polymerization shrinkage stress. The urethanized (meth)acrylic compound (b-3a) can be easily synthesized by, for example, an addition reaction of a polyol containing a polymer skeleton (described later), a compound having an isocyanate group (-NCO), and a (meth)acrylic compound having a hydroxyl group (-OH). Alternatively, the urethanized (meth)acrylic compound (b-3a) can be synthesized with ease by, for example, allowing lactone or alkylene oxide to undergo a ring-opening addition reaction with a (meth)acrylic compound having a hydroxyl group, and causing the resulting compound having a terminal hydroxyl group to undergo an addition reaction with a compound having an isocyanate group. The (meth)acrylic compound (b-3b) having no urethane skeleton can be obtained, for example, through a dehydrocondensation reaction of (meth)acrylic acid with a polymer of a monomer having a hydroxyl group.

· Urethanized (Meth)Acrylic Compound (b-3a)

The urethanized (meth)acrylic compound (b-3a) is preferably a (meth)acrylate having a structure (a polymer skeleton) selected from the group consisting of a polyester, a polycarbonate, a polyurethane, a polyether, a poly-conjugated diene, and a hydrogenated poly-conjugated diene, in addition to a urethane bond. More preferably, the urethanized (meth)acrylic compound (b-3a) is a (meth)acrylate having at least one polyol moiety, per molecule, selected from the group consisting of a polyester, a polycarbonate, a polyurethane, a polyether, a poly-conjugated diene, and a hydrogenated poly-conjugated diene each having a structure derived from a C4 to C18 aliphatic diol unit having a branched structure.

In such structures, examples of the polyester include: a copolymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a copolymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of δ-valerolactone; a polymer of ε-caprolactone; and a copolymer of these. Preferred are a copolymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a copolymer of a dicarboxylic acid (a saturated aliphatic dicarboxylic acid such as adipic acid or sebacic acid) and an aliphatic diol having 2 to 12 carbon atoms.

Examples of the polycarbonate include polycarbonates derived from an aliphatic diol having 2 to 18 carbon atoms, polycarbonates derived from bisphenol A, and polycarbonates derived from a C2 to C18 aliphatic diol and bisphenol A. Preferred are polycarbonates derived from an aliphatic diol having 2 to 12 carbon atoms, polycarbonates derived from bisphenol A, and polycarbonates derived from a C2 to C12 aliphatic diol and bisphenol A.

Examples of the polyurethane include a polymer of a C2 to C18 aliphatic diol and a C1 to C18 diisocyanate. Preferred is a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior mechanical strength and water resistance, preferred among these structures are polyesters, polycarbonates, and poly-conjugated dienes. A polyol having the polymer skeleton mentioned above can be used for the production of urethanized (meth)acrylic compound (b-3a).

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include:

hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylate of dipentaerythritol; and

hydroxy (meth)acrylamide compounds such as N-hydroxyethyl(meth)acrylamide, and N,N-bis(2-hydroxyethyl)(meth)acrylamide.

Examples of the C4 to C18 aliphatic diol having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a self-adhesive dental composite resin having superior curability, the polyol components used are preferably C5 to C12 aliphatic diols having a methyl-group side chain, for example, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol. The polyol components are more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

The addition reaction of the compound having an isocyanate group and the (meth)acrylic compound having a hydroxyl group can be performed following a known method, and the method is not particularly limited.

The urethanized (meth)acrylic compound (b-3a) obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; the compound having an isocyanate group; and the (meth)acrylic compound having a hydroxyl group.

· (Meth)Acrylic Compound (b-3b) Having no Urethane Skeleton

The (meth)acrylic compound (b-3b) having no urethane skeleton preferably has a structure (a polymer skeleton) selected from the group consisting of a polyester, a polycarbonate, a polyurethane, a polyether, a poly-conjugated diene, and a hydrogenated poly-conjugated diene.

In such structures, examples of the polyester include a copolymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a copolymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of δ-valerolactone; a polymer of ε-caprolactone; and a copolymer of these. Preferred are a copolymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a copolymer of a dicarboxylic acid (a saturated aliphatic dicarboxylic acid such as adipic acid or sebacic acid) and an aliphatic diol having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior flexibility and superior water resistance, preferred among these are the polyester, polycarbonate, and poly-conjugated diene structures. A polyol having the polymer skeleton mentioned above can be used for the production of (meth)acrylic compound (b-3b) having no urethane skeleton.

The glass transition temperature and acetone solubility of the (meth)acrylic compound (b-3b) having no urethane skeleton can be adjusted by adjusting the skeleton or molecular weight of a structure (polymer skeleton) selected from the group consisting of a polyester, a polycarbonate, a polyurethane, a polyether, a poly-conjugated diene, and a hydrogenated poly-conjugated diene.

Examples of the (meth)acrylic compound having a hydroxyl group include:

hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol, and

hydroxy (meth)acrylamide compounds such as N-hydroxyethyl (meth)acrylamide, and N,N-bis(2-hydroxyethyl)(meth)acrylamide.

The (meth)acrylic compound (b-3b) having no urethane skeleton obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; and the (meth)acrylic compound having a hydroxyl group.

In view of viscosity and the effect to reduce polymerization shrinkage stress, the (meth)acrylic compound (b-3) has a weight-average molecular weight (Mw) of preferably 1,000 to 80,000, more preferably 2,000 to 50,000, even more preferably 3,000 to 20,000. In the present invention, weight-average molecular weight (Mw) refers to a weight-average molecular weight measured by gel permeation chromatography (GPC) in terms of polystyrene.

The (meth)acrylic compound (b-3) has a weight-average molecular weight of preferably 1,250 or more and less than 20,000, more preferably 1,500 or more and 17,500 or less, even more preferably 1,800 or more and 16,000 or less, particularly preferably 2,500 or more and 15,000 or less per (meth)acryl group. With the number of polymerizable groups confined in these ranges in the (meth)acrylic compound (b-3a), crosslinkage takes place in a moderate fashion, and it is possible to more effectively maintain the mechanical strength while reducing polymerization shrinkage stress. When the (meth)acrylic compound (b-3) contains polymerizable groups other than (meth)acryl groups, for example, such as vinyl groups or styrene groups, the number of such polymerizable groups other than (meth)acryl groups in the (meth)acrylic compound (b-3) is preferably 2 or less, more preferably 0, because the presence of such polymerizable groups may lead to increased polymerization shrinkage stress depending on the form of polymerization.

In certain embodiments, the polarity difference between (meth)acrylic compound (b-3) and other monomer components is important for obtaining an even greater polymerization shrinkage stress reducing effect, and, to this end, it is preferable for the (meth)acrylic compound (b-3) to have a lower solubility in acetone relative to other monomers having a higher solubility in acetone. In view of the compatibility with other monomer components, the (meth)acrylic compound (b-3) has a solubility in acetone of preferably less than 50 g/L, more preferably less than 30 g/L, even more preferably less than 25 g/L, particularly preferably less than 20 g/L, most preferably less than 10 g/L.

A solubility parameter (SP) value may be used as a parameter that measures the difference in polarity and compatibility between (meth)acrylic compound (b-3) and other monomer components. The SP value is a measure of solubility. Higher SP values indicate higher polarity, and lower SP values indicate lower polarity. In view of the polymerization shrinkage stress reducing effect, the (meth)acrylic compound (b-3) and other monomer components have an SP value difference of preferably 1.0 $(cal/cm^3)^{1/2}$ or more, more preferably 2.0 $(cal/cm^3)^{1/2}$ or more, even more preferably 3.0 $(cal/cm^3)^{1/2}$ or more, particularly preferably 4.0 $(cal/cm^3)^{1/2}$ or more.

The SP value can be measured as follows. A sample is weighed into a 100 mL Erlenmeyer flask in an amount of 0.5 g, and 10 mL of acetone is added to dissolve the sample. Hexane is dropped into the mixture while stirring the mixture with a magnetic stirrer, and the volume of hexane (vh) that produced cloudiness in the solution (cloud point) is determined. Subsequently, the volume of deionized water (vd) at the cloud point is determined by adding deionized water instead of hexane. The SP value can then be determined from vh and vd using the formula presented in reference literature SUH, CLARKE, J. P. S. A-1, 5, 1671 to 1681 (1967). When this method cannot be used to find the SP value such as when the sample does not dissolve in acetone, the SP value can be estimated using the method proposed by Fedors et al. Specifically, the SP value can be determined by referring to Polymer Engineering and Science, February, 1974, Vol. 14, No. 2, ROBERF. FEDORS. (pp. 147 to 154). More specifically, the SP value can be calculated by the Fedors method using the following formula (A).

$$\text{SP value} = (\text{CED value})^{1/2} = (E/V)^{1/2} \qquad \text{Formula (A)}$$

In the formula (A), E is the cohesive energy (cal/mol), and V is the molar molecular volume ($cm^3$/mol).

The (meth)acrylic compound (b-3) may include at least one glass transition temperature (hereinafter, also referred to simply as "Tg") that is less than 40°C. Tg is not particularly limited, as long as it is less than 40°C. However, in view of providing an even greater polymerization shrinkage stress reducing effect, it is preferable that Tg fall in a temperature range of preferably -100°C to 30°C, more preferably -75°C to 15°C, even more preferably -60°C to 10°C. When the (meth)acrylic compound (b-3) solely has a Tg of 40°C or more, the (meth)acrylic compound (b-3) turns into a vitreous state at the temperature of polymerization, and fails to exhibit a sufficient polymerization shrinkage stress reducing effect. The (meth)acrylic compound (b-3) may have more than one Tg, as described below. In this case, one of the multiple glass transition temperatures Tg is less than 40°C, while the other glass transition temperatures Tg may be in a temperature range of 40°C or more. For example, the (meth)acrylic compound (b-3) includes a compound having two glass transition temperatures Tg, one at -42°C, and the other at 44.6°C. In certain embodiments, the (meth)acrylic compound (b-3) is preferably one having no aromatic ring in its skeleton, in order to prevent an increase of glass transition temperature, and adjust the glass transition temperature to less than 40°C. In other embodiments, the (meth)acrylic compound (b-3) is preferably one that does not have a cyclic structure (an aromatic ring, a heterocyclic ring, or an alicyclic structure) in its skeleton, in order to adjust the glass transition temperature to less than 40°C.

In the present invention, a glass transition temperature (Tg) means the midpoint glass transition temperature ($T_{mg}$). Specifically, a glass transition temperature (Tg) in the present invention can be determined by measurement based on JIS K 7121-1987 (rev. 2012). In the following, "baseline" means a DTA or DSC curve in a temperature range in which glass transitions or reactions do not occur in the measurement sample, as defined in JIS K 7121-1987 (rev. 2012). The midpoint glass transition temperature ($T_{mg}$) is the temperature where the straight line vertically equidistant from the straight line as an extension of each baseline intersects the curve where the glass transition shows a step-like change. FIG. 2 shows the extrapolated glass transition onset temperature ($T_{ig}$), extrapolated glass transition end temperature ($T_{eg}$), and midpoint glass transition temperature ($T_{mg}$). The extrapolated glass transition onset temperature ($T_{ig}$) is the temperature where a straight line as an extension of the baseline on the low-temperature side toward the high-temperature side intersects the tangent to the steepest slope of the curve where the glass transition shows a step-like change. The extrapolated glass transition end temperature ($T_{eg}$) is the temperature where the straight line as an extension of the baseline on the high-temperature side toward the low-temperature side intersects the tangent to the steepest slope of the curve where the glass transition shows a step-like change. In heat flux DSC, the extrapolated glass transition end temperature ($T_{eg}$) of when a peak appears on the high-temperature side of a step-like change is the temperature where the straight line as an extension of the baseline on the high-temperature side toward the low-temperature side intersects the tangent to the steepest slope of the curve on the high-temperature side of the peak.

In certain embodiments, the (meth)acrylic compound (b-3) preferably has two or more glass transition temperatures, more preferably two glass transition temperatures. In two or more glass transition temperatures Tg, it is preferable that one or more glass transition temperatures Tg be present on the low-temperature side and on the high-temperature side. In such embodiments, a first glass transition temperature Tg (on the low-temperature side) is preferably -100°C or more and 20°C or less, more preferably -75°C or more and 15°C or less, even more preferably -60°C or more and 10°C or less. In such embodiments, a second glass transition temperature Tg (on the high-temperature side) is preferably 20°C or more and less than 80°C, more preferably 25°C or more and less than 70°C, even more preferably 30°C or more and less than 65°C. A certain embodiment is, for example, a self-adhesive dental composite resin in which the (meth) acrylic compound (b-3) has one or more glass transition temperatures in the temperature region of -100°C or more and 20°C or less, and one or more glass transition temperatures in the temperature region of 20°C or more and less than 80°C.

In view of handling properties and the polymerization shrinkage stress reducing effect, the (meth)acrylic compound (b-3) has a viscosity at 25°C of preferably 1,000 to 10,000,000 mPa·s, more preferably 5,000 to 7,500,000 mPa·s, even more preferably 10,000 to 7,000,000 mPa·s. In the present invention, viscosity means a viscosity measured at 25°C with a Brookfield rotary viscometer. Measurement conditions, such as time and rotational speed, are appropriately adjusted according to the viscosity range.

The (meth)acrylic compound (b-3) may be a commercially available product. Examples of such commercially available products include urethane polymers having a terminal polymerizable group, such as the Art Resin series (UN-7600, UN7700) manufactured by Negami Chemical Industrial Co., Ltd.; the Kuraprene series (polyisoprene skeleton or polybutadiene skeleton) manufactured by Kuraray Co., Ltd. (LIR-30, LIR-50, LIR-390, LIR-403, LIR-410, UC-102M, UC-203M, LIR-700, LBR-302, LBR-307, LBR-305, LBR-352, LBR-361, L-SBR-820, L-SBR-841); polyols manufactured by Kuraray Co., Ltd. (P-6010, P-5010, P-4010, P-3010, P-2010, P-1010, F-3010, F2010, F-1010, P-2011, P-1020, P-2020, P-530, P-2030, P-2050, C-2090); and the liquid polybutadiene NISSO-PB manufactured by Nippon Soda Co., Ltd. (B-1000, B-2000, B-3000, BI-2000, BI-3000, G-1000, G-2000, G-3000, GI-1000, GI-2000, GI-3000, TEAI-1000, TE-2000, TE-4000, JP-100, JP-200). Preferred are UN-7600, UN-7700, LBR-302, LBR-307, LBR-305, LBR-352, LBR-361, L-SBR-820, UC-102M, UC-203M, C-2090, P-2020, P-2050, B3000, BI-2000, BI-3000, TEAI-1000, TE-2000, and TE-4000. More preferred are UN-7600, UN-7700, UC-102M, TE-2000, and TE-4000.

In view of mechanical strength, paste properties, and polymerization shrinkage stress reducing effect, the

content of the (meth)acrylic compound (b-3) in a self-adhesive dental composite resin of the present invention is preferably 0.1 to 50 parts by mass, more preferably 0.5 to 40 parts by mass, even more preferably 1 to 35 parts by mass, particularly preferably 1 to 25 parts by mass, most preferably 1 to 18 parts by mass with respect to total 100 parts by mass of the monomer components. In certain embodiments, in view of mechanical strength, paste properties, and polymerization shrinkage stress reducing effect, the content of (meth)acrylic compound (b-3) is preferably 0.1 mass% or more, more preferably 0.5 mass% or more, even more preferably 1 mass% or more in the total mass of the self-adhesive dental composite resin. In view of mechanical strength, paste properties, and polymerization shrinkage stress reducing effect, the content of the (meth)acrylic compound (b-3) is preferably 30 mass% or less, more preferably 25 mass% or less, even more preferably 20 mass% or less in the total mass of the self-adhesive dental composite resin.

In view of the properties of the self-adhesive dental composite resin, and superior cavity sealing properties (particularly, cavity sealing properties in wedge-shaped defect cavities), as well as durability of cavity sealing properties, the content of the monomer (b) having no acidic group in the self-adhesive dental composite resin is preferably 60 to 99 parts by mass, more preferably 65 to 97.5 parts by mass, even more preferably 70 to 95 parts by mass in total 100 parts by mass of the monomers. In view of adhesive properties to tooth structure and the water absorption and solubility of the cured product, the mass ratio of hydrophobic monomer (b-1) to hydrophilic monomer (b-2) is preferably 1:0 to 1:2, more preferably 1:0 to 1:1, even more preferably 1:0 to 2:1.

<Polymerization Initiator (c)>

The polymerization initiator (c) is an essential component for curing the monomers. The polymerization initiator (c) may be a photopolymerization initiator (c-1) or chemical polymerization initiator (c-2). The polymerization initiator (c) may be incorporated alone, or two or more thereof may be used in combination.

The photopolymerization initiator (c-1) can be classified into water-soluble photopolymerization initiator (c-1a) and water-insoluble photopolymerization initiator (c-1b). The photopolymerization initiator (c-1) may be solely a water-soluble photopolymerization initiator (c-1a) or a water-insoluble photopolymerization initiator (c-1b), or may be a combination of a water-soluble photopolymerization initiator (c-1a) and a water-insoluble photopolymerization initiator (c-1b). Preferably, a water-soluble photopolymerization initiator (c-1a) and a water-insoluble photopolymerization initiator (c-1b) are used in combination.

· Water-Soluble Photopolymerization Initiator (c-1a)

The water-soluble photopolymerization initiator (c-1a) improves the polymerization curability at the interface with hydrophilic tooth surfaces, resulting in high bond strength and contributing to superior cavity sealing properties (particularly, cavity sealing properties in wedge-shaped defect cavities) and the durability of cavity sealing properties. The water-soluble photopolymerization initiator (c-1a) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (c-1a) can sufficiently dissolve in water within the tooth structure at the bonding interface, and more easily exhibits the polymerization promoting effect.

Examples of the water-soluble photopolymerization initiator (c-1a) include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having $-OCH_2COO^-Na^+$ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having $-OCH_2COO^-Na^+$ introduced into the phenyl group of 2-hydroxy-2-methyl-1-phenylpropan-1-one. Other examples of the water-soluble photopolymerization initiator (c-1a) include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1-[(4-morpholino)phenyl]-1-butanon.

The water-soluble thioxanthones may be any of, for example,

2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminiu m chloride,
2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneami nium chloride,
2-hydroxy-3-(3,4-dimethyl-9H-thioxanthen-2-yloxy)-N,N,N-trimethyl-1-propaneaminium chloride, and
2-hydroxy-3-(1,3,4-trimethyl-9-oxo-9H-thioxanthen-2-yloxy)-N,N,N-trimethyl-1-propanea minium chloride.

Examples of the water-soluble acylphosphine oxides include acylphosphine oxides represented by the following general formula (1), (2), or (3).

[Chem. 1]

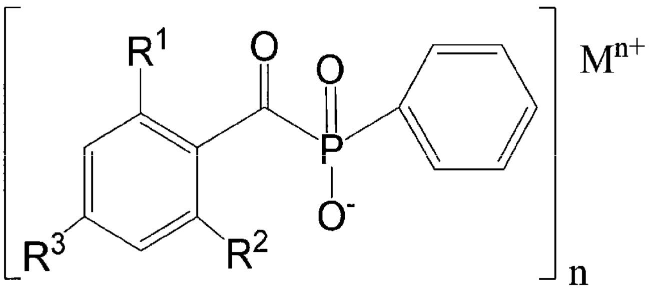

(1)

[Chem. 2]

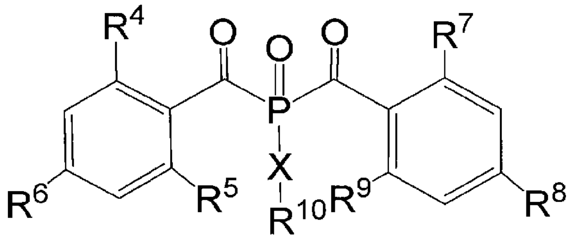

(2)

[Chem. 3]

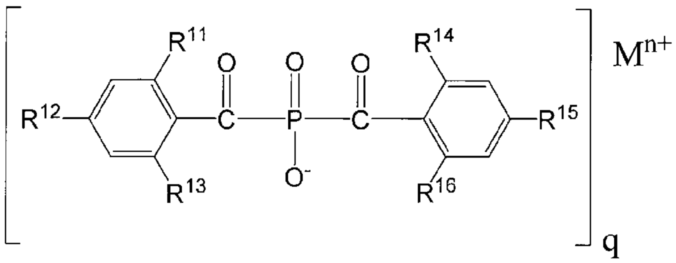

(3)

In formulae (1), (2), and (3), $R^1$ to $R^9$ and $R^{11}$ to $R^{16}$ are each independently a $C_1$ to $C_4$ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by $HN^+R^{17}R^{18}R^{19}$ (where $R^{17}$, $R^{18}$, and $R^{19}$ are each independently an organic group or a hydrogen atom), n and q are each 1 or 2, X is a $C_1$ to $C_4$ linear or branched alkylene group, and $R^{10}$ represents $-CH(CH_3)COO(C_2H_4O)_pCH_3$, where p represents an integer of 1 to 1,000.

The alkyl groups represented by $R^1$ to $R^9$ and $R^{11}$ to $R^{16}$ are not particularly limited, as long as these are $C_1$ to $C_4$ linear or branched alkyl groups. Examples include methyl groups, ethyl groups, n-propyl groups, isopropyl groups, n-butyl groups, isobutyl groups, sec-butyl groups, 2-methylpropyl groups, and tert-butyl groups. The alkyl groups represented by $R^1$ to $R^9$ are preferably $C_1$ to $C_3$ linear alkyl groups, more preferably methyl or ethyl groups, even more preferably methyl groups. Examples of X include methylene groups, ethylene groups, n-propylene groups, isopropylene groups, and n-butylene groups. X is preferably a $C_1$ to $C_3$ linear alkylene group, more preferably a methylene or ethylene group, even more preferably a methylene group.

Examples of the substituent of the pyridine ring when M is a pyridinium ion include halogen atoms (a fluorine atom, a chlorine atom, a bromine atom, an iodine atom), carboxyl groups, $C_2$ to $C_6$ linear or branched acyl groups, $C_1$ to $C_6$ linear or branched alkyl groups, and $C_1$ to $C_6$ linear or branched alkoxy groups. Preferably, M is an alkali metal ion, an alkali earth metal ion, a magnesium ion, a pyridinium ion (the pyridine ring may have a substituent), or an ammonium ion represented by $HN^+R^{17}R^{18}R^{19}$ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic groups represented by $R^{17}$, $R^{18}$, and $R^{19}$ may be

the same groups exemplified above for the substituent of the pyridine ring (excluding a halogen atom).

In view of storage stability and shade stability in a composition of the self-adhesive dental composite resin, particularly preferred are compounds in which $R^1$ to $R^3$ are all methyl groups in general formula (1), compounds in which $R^4$ to $R^9$ are all methyl groups in general formula (2), and compounds in which $R^{11}$ to $R^{16}$ are all methyl groups in general formula (3). Examples of ammonium ions include ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in $R^{10}$ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferred among these water-soluble acylphosphine oxides are sodium phenyl(2,4,6-trimethylbenzoyl)phosphinate, lithium phenyl(2,4,6-trimethylbenzoyl)phosphinate, sodium bis(2,4,6-trimethylbenzoyl)phosphinate, lithium bis(2,4,6-trimethylbenzoyl)phosphinate, and compounds represented by general formula (2) and in which the moiety corresponding to the group represented by $R^{10}$ is synthesized from polyethylene glycol methyl ether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (c-1a) may be used alone, or two or more thereof may be used in combination.

The water-soluble photopolymerization initiator (c-1a) may be dissolved in the self-adhesive dental composite resin, or may be dispersed in the form of a powder in a composition of the self-adhesive dental composite resin.

When the water-soluble photopolymerization initiator (c-1a) is dispersed in the form of a powder in a composition of the self-adhesive dental composite resin, the average particle diameter of the powder is preferably 500 μm or less, more preferably 100 μm or less, even more preferably 50 μm or less because the water-soluble photopolymerization initiator (c-1a) tends to settle when the powder has an excessively large average particle diameter. The average particle diameter is preferably 0.01 μm or more because the specific surface area of the powder overly increases, and the amount of powder that can be dispersed in a composition of the self-adhesive dental composite resin decreases when the powder has an excessively small average particle diameter. Taken together, the average particle diameter of water-soluble photopolymerization initiator (c-1a) preferably ranges from 0.01 to 500 μm, more preferably 0.01 to 100 μm, even more preferably 0.01 to 50 μm.

The average particle diameter of a powder of individual water-soluble photopolymerization initiators (c-1a) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The shape of the water-soluble photopolymerization initiator (c-1a) when it is dispersed in powder form is not particularly limited, and may be any of various shapes, including, for example, spherical, stylus, plate-like, and crushed shapes. The water-soluble photopolymerization initiator (c-1a) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the self-adhesive dental composite resin obtained, the content of water-soluble photopolymerization initiator (c-1a) is preferably 0.01 to 20 parts by mass relative to total 100 parts by mass of monomers in a self-adhesive dental composite resin of the present invention. In view of adhesive properties to tooth structure, the content of water-soluble photopolymerization initiator (c-1a) is more preferably 0.05 to 10 parts by mass, even more preferably 0.1 to 5 parts by mass relative to total 100 parts by mass of monomers in the self-adhesive dental composite resin. When the content of water-soluble photopolymerization initiator (c-1a) is at or above these lower limits, polymerization can sufficiently proceed at the bonding interface, making it easier to provide a sufficient bond strength. When the content of water-soluble photopolymerization initiator (c-1a) is at or below the foregoing upper limits, it is easier to provide a sufficient bond strength.

· Water-Insoluble Photopolymerization Initiator (c-1b)

In view of curability and mechanical strength, the self-adhesive dental composite resin preferably comprises a water-insoluble photopolymerization initiator (c-1b) having a solubility of less than 10 g/L in 25°C water (hereinafter, also referred to as "water-insoluble photopolymerization initiator (c-1b)"), in addition to the water-soluble photopolymerization initiator (c-1a). The water-insoluble photopolymerization initiator (c-1b) used in the present invention may be a known photopolymerization initiator. The water-insoluble photopolymerization initiator (c-1b) may be incorporated alone, or two or more thereof may be incorporated in combination.

Examples of the water-insoluble photopolymerization initiator (c-1b) include (bis)acylphosphine oxides (other than those exemplified for the water-soluble photopolymerization initiator (c-1a)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene) acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethy l-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (c-1b) is preferably at least one selected from the group consisting of a (bis)acylphosphine oxide, an α-diketone, and a coumarin. In this way, a self-adhesive dental composite resin can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (c-1b) is not particularly limited. However, in view of curability and other properties of the composition of self-adhesive dental composite resin obtained, the content of water-insoluble photopolymerization initiator (c-1b) is preferably 0.01 to 10 parts by mass, more preferably 0.05 to 7 parts by mass, even more preferably 0.1 to 5 parts by mass relative to total 100 parts by mass of monomers in the self-adhesive dental composite resin.

When the content of water-insoluble photopolymerization initiator (c-1b) is at or below these upper limits, it is easier to obtain a sufficient bond strength even when the water-insoluble photopolymerization initiator (c-1b) itself has low polymerization performance, in addition to reducing precipitation of the water-insoluble photopolymerization initiator (c-1b) itself from the self-adhesive dental composite resin.

When the water-soluble photopolymerization initiator (c-1a) and the water-insoluble photopolymerization initiator (c-1b) are used in combination, the mass ratio (c-1a):(c-1b) of water-soluble photopolymerization initiator (c-1a) and water-insoluble photopolymerization initiator (c-1b) in the present invention is preferably 10:1 to 1:10, more preferably 7:1 to 1:7, even more preferably 5:1 to 1:5, particularly preferably 3:1 to 1:3. When the fraction of water-soluble photopolymerization initiator (c-1a) in the mass ratio exceeds 10:1, it may lead to a decrease in bond strength to tooth structure or flexural strength due to a decrease in the curability of the self-adhesive dental composite resin itself, potentially affecting the intended effects of the present invention, such as favorable cavity sealing properties (particularly, cavity sealing properties in wedge-shaped defect cavities) and the durability of cavity sealing properties. When the fraction of water-insoluble photopolymerization initiator (c-1b) in the mass ratio exceeds 1:10, the self-adhesive dental composite resin may have difficulty in exhibiting high bond strength as a result of insufficient promotion of polymerization at the bonding interface, though the curability of the composition itself can increase.

· Chemical Polymerization Initiator (c-2)

In view of enabling chemical polymerization and improving cavity sealing properties in areas with no access to light, the self-adhesive dental composite resin may additionally comprise a chemical polymerization initiator (c-2). Preferred for use as chemical polymerization initiator (c-2) are organic peroxides. The organic peroxides used as chemical polymerization initiator (c-2) are not particularly limited, and known organic peroxides may be used. Typical examples of such organic peroxides include ketone peroxides, hydroperoxides, diacyl peroxides, dialkyl peroxides, peroxyketals, peroxyesters, and peroxydicarbonates. Specific examples of these organic peroxides include those mentioned in WO2008/087977. The chemical polymerization initiator may be used alone, or two or more thereof may be used in combination.

<Filler (d)>

The self-adhesive dental composite resin comprises a filler (d) to adjust handling properties, while also aiming to enhance the mechanical strength (such as flexural strength) of the cured product, provide superior shade conformity to both enamel and dentin, and reduce water absorption and dissolution. Examples of such fillers include inorganic fillers, organic-inorganic composite fillers, and organic fillers. The filler (d) may be used alone, or two or more thereof may be used in combination.

The material of inorganic fillers preferably contain various types of glass primarily composed of silica (with a silica content of 5 mass% or more, preferably 10 mass% or more), with optional oxides of elements such as heavy metals, boron, and aluminum.

Examples of the inorganic fillers include glass powders of common compositions, such as fused silica, quartz, soda-lime-silica glass, E glass, C glass, and borosilicate glass (PYREX® glass). Other examples include barium glass, strontium·borosilicate glass, lanthanum glass-ceramics, fluoroaluminosilicate glass, various ceramics, composite oxides (such as alumina, silica-titania, silica-zirconia, ytterbium oxide, silica-coated ytterbium fluoride, zirconia-coated silica, aluminosilicate glass, barium boroaluminosilicate glass, calcium fluoroaluminosilicate glass, strontium fluoroaluminosilicate glass, barium fluoroaluminosilicate glass, and strontium calcium fluoroaluminosilicate glass), diatomaceous earth, kaolin, clay minerals (such as montmorillonite), activated earth, synthetic zeolite, mica, calcium fluoride, ytterbium fluoride, yttrium fluoride, calcium phosphate, barium sulfate, zirconium oxide, titanium oxide, and hydroxyapatite. These may be used alone, or two or more thereof may be used in combination. The inorganic fillers may contain a silica atom, a zirconium atom, a titanium atom, or an aluminum atom coating the surface of these various types of glass.

A certain embodiment is, for example, a self-adhesive dental composite resin in which the filler (d) is a filler surface-treated with a surface treatment agent that does not contain zirconium atoms as atoms that coat the surface of various types of glass.

Preferred among these are inorganic fillers composed of metallic elements that provide high radiopacity, such as aluminum, strontium, zirconium, barium, lanthanum, ytterbium, titanium, and bismuth (for example, inorganic fillers such as barium glass, alumina, silica-titania, silica-zirconia, and silica-coated ytterbium fluoride). The inorganic fillers may be used as inorganic fillers with a relatively high refractive index. These may be used alone, or two or more thereof may be used as a mixture.

In view of providing superior mechanical strength and transparency in the self-adhesive dental composite resin obtained, preferred are quartz, silica, silica-titania, silica-zirconia, barium glass, ytterbium oxide, and silica-coated ytterbium fluoride, more preferably quartz, silica, silica-titania, silica-zirconia, barium glass, and silica-coated ytterbium fluoride.

In view of the radiopacity of the self-adhesive dental composite resin, preferred are silica-zirconia, barium glass, and silica-coated ytterbium fluoride. Silica-coated ytterbium fluoride is particularly preferred in view of storage stability.

In view of considerations such as the handling properties and mechanical strength of the self-adhesive dental

composite resin obtained, the inorganic fillers have an average particle diameter of preferably 0.001 to 50 μm, more preferably 0.001 to 20 μm, even more preferably 0.005 to 10 μm. In the present invention, the average particle diameter of inorganic fillers means the average particle diameter before surface treatment when inorganic fillers are subjected to surface treatment, as will be described. A certain preferred embodiment is, for example, a self-adhesive dental composite resin in which the filler (d) is an inorganic filler.

The inorganic fillers may be commercially available products. Examples of such commercially available products include silica such as Aerosil® 90, Aerosil® 130, Aerosil® 150, Aerosil® 200, Aerosil® 255, Aerosil® 300, Aerosil® 380, Aerosil® OX50, and Aerosil® R972 (all manufactured by Nippon Aerosil Co., Ltd.), GM27884, 8235 (manufactured by Schott), barium glass such as E-3000 (product code; manufactured by Esstech), strontium·borosilicate glass (E-4000, manufactured by ESSTECH), lanthanum glass-ceramics (GM31684, manufactured by Schott), and fluoroaluminosilicate glass (GM35429, G018-091, and G018-117, manufactured by Schott).

The inorganic fillers may be amorphous or crystalline, or a combination of these. However, the inorganic fillers are preferably at least partially amorphous.

The shape of inorganic fillers is not particularly limited, and the particle diameter of fillers may be appropriately selected for use. Examples include irregularly shaped fillers (crushed fillers) and spherical fillers. In view of improving the mechanical strength and degree of light diffusion D of the cured product of the self-adhesive dental composite resin, preferred for use as inorganic fillers are irregularly shaped fillers. Spherical fillers are preferred in view of handling properties. Here, spherical fillers represent particles that appear round in shape when observed in a unit field of an electron micrograph, and that have an average uniformity of 0.6 or more as calculated by dividing the diameter of a particle perpendicular to its maximum diameter by the maximum diameter.

In order to adjust the mechanical strength and flowability of the self-adhesive dental composite resin, it is preferable to use the inorganic fillers after a surface treatment with a known surface treatment agent such as a silane coupling agent. For example, the hydroxyl groups present on the surface of inorganic fillers may be surface treated with a surface treatment to obtain inorganic fillers having its hydroxyl groups surface treated.

Examples of the surface treatment agent include silane coupling agents such as vinyltrimethoxysilane, vinyltriethoxysilane, vinyltrichlorosilane, vinyltri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane. Preferred are vinyltrimethoxysilane, γ-methacryloyloxypropyltrimethoxysilane, 8-methacryloyloxyoctyltrimethoxysilane, 11-methacryloyloxyundecyltrimethoxysilane, γ-aminopropyltriethoxysilane; and nanosilica.

The surface treatment can be carried out using any known method, including, for example, a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the surface of inorganic filler and the surface treatment agent can proceed to completion to enable a surface treatment by applying heat in a temperature range of typically 50 to 150°C. The amount of surface treatment is not particularly limited. For example, the amount of surface treatment agent may be 0.1 to 40 parts by mass relative to 100 parts by mass of inorganic filler before surface treatment.

The organic-inorganic composite fillers are fillers obtained by adding a monomer to the inorganic filler, polymerizing the mixture in paste form, and pulverizing the polymerized filler. The organic-inorganic composite filler refers to a filler comprising the inorganic filler and a polymer of a monomer. The organic-inorganic composite filler may be, for example, a filler obtained by mixing Bis-GMA, 3G, and a surface-treated silica filler, and pulverizing the mixture after polymerization. The shape of the organic-inorganic composite filler is not particularly limited, and the particle diameter of fillers may be appropriately selected for use. The organic-inorganic composite fillers may be used alone, or two or more thereof may be used as a mixture. In view of mechanical strength, the organic-inorganic composite fillers are preferably surface-treated. Examples of surface treatment agents and the preferred varieties of surface treatment agents are no different from those described in conjunction with inorganic fillers. In view of properties such as the handling properties and mechanical strength of the composition of self-adhesive dental composite resin obtained, the organic-inorganic composite fillers have an average particle diameter of preferably 0.001 to 50 μm, more preferably 0.001 to 20 μm, even more preferably 0.005 to 15 μm.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of organic fillers is not particularly limited, and the particle diameter of fillers may be appropriately selected for use. In view of considerations such as the handling properties and mechanical strength of the self-adhesive dental composite resin obtained, the average particle

diameter of the organic fillers is preferably 0.001 to 50 μm, more preferably 0.001 to 20 μm, even more preferably 0.005 to 15 μm.

In this specification, the average particle diameter of filler can be determined by a laser diffraction scattering method or by observing particles with an electron microscope. Specifically, a laser diffraction scattering method is more convenient for the measurement of particles having a particle size of 0.1 μm or more, whereas electron microscopy is a more convenient method of particle size measurement for ultrafine particles of less than 0.1 μm. Here, 0.1 μm is a measured value by a laser diffraction scattering method. In particles such as agglomerated particles formed by primary particles, there are average particle diameters for primary particles and secondary particles. In such cases, the average particle diameter of fillers refers to the average particle diameter of the larger secondary particles.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average particle diameter is calculated from the number of particles and the particle diameter.

In the self-adhesive dental composite resin, it is preferable to use a mixture or a combination of two or more fillers of different materials, different particle size distributions, and different forms.

By combining two or more types of fillers, the fillers can interact with the monomer or with the other fillers at an increased number of points, in addition to densely packing themselves. Depending on the type of filler, it is also possible to control paste flowability in the presence or absence of a shear force. In view of the handling and paste properties of the self-adhesive dental composite resin, the filler (d) is preferably a combination (I) of a filler (d-i) having an average particle diameter of 1 nm or more and less than 0.1 μm (hereinafter, also referred to as "filler (d-i)") and a filler (d-ii) having an average particle diameter of 0.1 μm or more and 1 μm or less (hereinafter, also referred to as "filler (d-ii)"); a combination (II) of filler (d-i) and a filler (d-iii) having an average particle diameter of more than 1 μm and 10 μm or less (hereinafter, also referred to as "filler (d-iii)"); a combination (III) of filler (d-i), filler (d-ii), and filler (d-iii); a combination (IV) of two or more fillers (d-ii); a combination (V) of two or more fillers (d-iii) having an average particle diameter of more than 1 μm and 10 μm or less; or a combination (VI) of filler (d-i) and two or more fillers (d-iii). In view of paste properties and cavity sealing properties (particularly, cavity sealing properties in wedge-shaped defect cavities) and the durability of cavity sealing properties, more preferred are (I), (II), (III), (V), and (VI), even more preferably (II), (III), (V), and (VI).

In view of cavity sealing properties (particularly, cavity sealing properties in wedge-shaped defect cavities) and the durability of cavity sealing properties, as well as shade conformity to both enamel and dentin, it is also possible to use three types of fillers, for example, a combination (VI) of a filler (d-i) having an average particle diameter of 1 nm or more and less than 0.1 μm, and two types of fillers (d-iii). In view of paste properties and handling, the combination (VI) is preferred in certain embodiments.

The combination (IV) of two or more types of fillers (d-ii) means an embodiment that comprises two or more types of fillers (d-ii) of different average particle diameters between 0.1 μm and 1 μm.

Similarly, the combination (IV) of two or more types of fillers (d-iii) having an average particle diameter of more than 1 μm and 10 μm or less means an embodiment that comprises two or more types of fillers (d-iii) of different average particle diameters larger than 1 μm and not exceeding 10 μm.

The fillers (d) of different particle diameters may include other types of fillers, provided that the fillers (d) have the foregoing combinations. Unintended inclusion of non-filler particles as impurities is acceptable to such an extent that it does not hinder the effectiveness of the present invention.

The content of filler (d-iii) is not particularly limited; however, in view of handling properties, the mechanical strength and light diffusing properties of the cured product, and cavity sealing properties (particularly, cavity sealing properties in wedge-shaped defect cavities), as well as the durability of cavity sealing properties, it is preferable in certain embodiments that the content of filler (d-iii) be 45 to 85 mass%, more preferably 50 to 80 mass%, even more preferably 55 to 75 mass% in total 100 mass% of the self-adhesive dental composite resin.

The same applies to the content of filler (d-ii).

In other certain embodiments, the filler (d-ii) may have an average particle diameter of more than 0.4 μm and 1 μm or less, taking into account its influence on visible light transmissivity, refractive index difference, and degree of light diffusion D.

The refractive index of filler (d) is not particularly limited, and may be, for example, 1.35 to 2.00. By bringing the refractive index of filler (d) closer to that of the other non-filler components in the cured product of the self-adhesive dental composite resin, it is easier to increase transparency in the cured product obtained. From an aesthetic standpoint where the cured product, as a dental restoration material, should be prevented from appearing unnaturally white compared to natural teeth, it is preferable to provide transparency in the cured product through the approximation of refractive indices. In

this respect, the refractive index of filler (d) is preferably 1.40 to 1.70, more preferably 1.45 to 1.65, even more preferably 1.50 to 1.60.

The refractive index of filler (d) can be controlled by, for example, adjusting the type and/or proportion of its constituents. For example, the refractive index of filler (d) can be increased when the filler (d) contains metallic elements such as aluminum, strontium, zirconium, barium, lanthanum, ytterbium, titanium, and bismuth as its constituents. The type and proportion of the constituents are not particularly limited, and may be altered according to the desired refractive index.

The refractive index can also be adjusted by combining a filler (d) having a low refractive index and a filler (d) having a high refractive index. Alternatively, the refractive index can be adjusted based on the crystallinity of filler (d) (crystalline or amorphous). The crystallinity can be appropriately adjusted using known methods.

In view of light diffusing properties, a certain preferred embodiment is, for example, a self-adhesive dental composite resin in which the filler (d) comprises two or more types of fillers with different types and/or proportions of constituents.

In one aspect, the filler (d) comprising two or more types of fillers with different types of constituents is, for example, a filler (d) in which one of the fillers is composed primarily of $SiO_2$, and another filler is composed primarily of $YbF_3$.

In another aspect, the filler (d) comprising two or more types of fillers with different proportions of constituents is, for example, a filler (d) in which two of the fillers both comprises $SiO_2$, and in which the absolute value of the difference between the proportions of $SiO_2$ (mass%) is different when their proportions are compared between the two fillers. The absolute value of the difference is preferably 1 or more, more preferably 5 or more, even more preferably 10 or more. When three or more types of fillers are present, the type and/or proportion of the constituents should be different between two certain types of fillers within these fillers.

Examples of the constituting elements of the constituents of the filler (d) include silicon, aluminum, strontium, zirconium, barium, lanthanum, ytterbium, titanium, and bismuth. It is preferable to comprise metallic elements such as aluminum, strontium, zirconium, barium, lanthanum, ytterbium, titanium, and bismuth, in addition to silicon.

Another certain preferred embodiment is, for example, a self-adhesive dental composite resin in which the constituting elements of the constituents of the filler (d) comprise silicon, and at least one selected from the group consisting of aluminum, strontium, zirconium, barium, lanthanum, ytterbium, titanium, and bismuth.

In certain embodiments, in view of shade conformity to both enamel and dentin, the proportions of the constituting elements (preferably, metals) of the constituents of the filler (d) are preferably 5 mass% or more, more preferably 7 mass% or more, even more preferably 10 mass% or more, and are preferably 90 mass% or less, more preferably 85 mass% or less, even more preferably 80 mass% or less, though these are not particularly limited.

In view of handling properties, it is preferable in another certain embodiment that the content of filler (d-i) be 10 mass% or less, more preferably 8 mass% or less, even more preferably 5 mass% or less in the total mass of the self-adhesive dental composite resin. The influence of filler (d-i) can be disregarded because the filler (d-i) has a lower proportion compared to other fillers, and has small overall impact on the self-adhesive dental composite resin in terms of its refractive index concerning shade conformity to both enamel and dentin.

In this specification, the refractive index of substances under measurement (such as filler (d) and colorants) can be measured with an Abbe refractometer. In the case of filler (d), its refractive index can be measured according to JIS K 0062:1992 with some modification, specifically, by a liquid immersion method at 25°C using an Abbe refractometer, with the sodium D-line serving as the light source. As for the liquid, different liquids with varying refractive indices are prepared by combining two or more liquids whose refractive indices are similar to the presumed refractive index of the sample filler. The sample is suspended in each liquid in a 25°C atmosphere, and the liquid appearing most transparent as observed by the naked eye is selected. By regarding the refractive index of this liquid as the refractive index of the sample, the liquid's refractive index is measured with an Abbe refractometer. Examples of usable liquids include diiodomethane with sulfur dissolved in it, 1-bromonaphthalene, methyl salicylate, dimethylformamide, and 1-pentanol.

As for the specific method of measuring the refractive index of monomer components, the refractive index can be measured in a 23°C atmosphere using an Abbe refractometer with the sodium D-line serving as the light source, in compliance with JIS K 0062:1992. For uncured monomer components, measurements can be taken in their liquid state. **In** the case of cured monomer components, these are measured after being formed into a certain size of cured plate. Examples of usable liquids include diiodomethane with sulfur dissolved in it, 1-bromonaphthalene, methyl salicylate, dimethylformamide, and 1-pentanol.

Including two or more types of fillers with different types and/or proportions of constituents allows for the incorporation of multiple fillers with different refractive indices, making it possible to impart light diffusing properties to the self-adhesive dental composite resin obtained. In view of light diffusing properties and favorable shade conformity to both enamel and dentin, the refractive index difference between these multiple fillers is preferably 0.02 or more, more preferably 0.03 or more, even more preferably 0.04 or more. In view of more readily achieving favorable shade conformity to both enamel and dentin of significantly different shades, the refractive index difference between multiple fillers is preferably less than 0.10, more preferably 0.09 or less, even more preferably 0.08 or less.

In this specification, the refractive index difference between fillers in an embodiment with multiple fillers (d) means the difference between the maximum and minimum values of refractive index, for example, when three or more types of fillers are incorporated.

In view of more readily achieving favorable shade conformity matching both enamel and dentin, a certain preferred embodiment is, for example, a self-adhesive dental composite resin in which the filler (d) has an average particle diameter of 0.1 μm or more, and comprises two or more fillers with different refractive indices.

In such embodiments, the refractive index difference between fillers (d) with different refractive indices is preferably 0.02 or more, more preferably 0.03 or more, even more preferably 0.04 or more. In view of more readily achieving favorable shade conformity matching both enamel and dentin of significantly different shades, the refractive index difference between these fillers (d) is preferably less than 0.10, more preferably 0.09 or less, even more preferably 0.08 or less.

In these embodiments, the fillers (d) with different refractive indices have an average particle diameter of preferably 25 μm or less, more preferably 20 μm or less, even more preferably 18 μm or less, particularly preferably 15 μm or less.

In these embodiments, the fillers (d) with different refractive indices have an average particle diameter of preferably 0.2 μm or more, more preferably 0.3 μm or more, even more preferably 0.4 μm or more, particularly preferably 0.5 μm or more.

In these embodiments, one of the fillers in the fillers (d) with different refractive indices has an average particle diameter of preferably more than 1.0 μm, more preferably 1.1 μm or more, even more preferably 1.2 μm or more. This filler may be an inorganic filler or an organic-inorganic composite filler, preferably an inorganic filler.

When an organic-inorganic composite filler is incorporated in these embodiments, the content of the organic-inorganic composite filler is preferably less than 30 mass%, more preferably 20 mass% or less, even more preferably 10 mass% or less in total 100 mass% of the self-adhesive dental composite resin.

When an inorganic filler is incorporated in these embodiments, the content of the inorganic filler is preferably 70 mass% or more, more preferably 80 mass% or more, even more preferably 90 mass% or more in total 100 mass% of filler (d).

In these embodiments, the fillers (d) with different refractive indices have a refractive index of preferably 1.40 to 1.70, more preferably 1.45 to 1.65, even more preferably 1.50 to 1.60.

In these embodiments, it is possible to achieve favorable shade conformity even when the constituents are of the same type and have the same proportions (for example, the filler has incorporated only $SiO_2$ and the $SiO_2$ content is 100%).

In these embodiments, the self-adhesive dental composite resin may comprise two or more types of fillers with different types and/or proportions of constituents.

The mixing ratio of two fillers with different types and/or proportions of constituents is not particularly limited, and is preferably 20:1 to 1:20, more preferably 10:1 to 1:10, even more preferably 1:6 to 6:1 by mass.

Another certain preferred embodiment is, for example, a self-adhesive dental composite resin in which one or more fillers with different refractive indices comprise at least one metallic element selected from the group consisting of aluminum, strontium, zirconium, barium, lanthanum, ytterbium, titanium, and bismuth.

By imparting a certain level of light diffusing properties to the cured product, the filling in wedge-shaped defect cavities in natural teeth becomes blurred in its margin (boundary) with natural teeth when the self-adhesive dental composite resin is filled into these cavities for restoration, making the filled area less noticeable. This results in favorable shade conformity matching both enamel and dentin, providing a highly aesthetic restoration method.

With light diffusing properties below certain levels, it is possible to impart a certain level of transparency to the self-adhesive dental composite resin obtained. By imparting a certain level of transparency, the shade of natural teeth at the cavity floor can be reflected in the filled area when the self-adhesive dental composite resin obtained is used to restore wedge-shaped defect cavities in natural teeth.

The content of filler (d) is not particularly limited. However, in view of handling properties, the mechanical strength and light diffusing properties of the cured product, and cavity sealing properties (particularly, cavity sealing properties in wedge-shaped defect cavities) and the durability of cavity sealing properties, the content of filler (d) is preferably 50 to 90 mass%, more preferably 55 to 85 mass%, even more preferably 60 to 80 mass% in total 100 mass% of the self-adhesive dental composite resin.

<Polymerization Accelerator (e)>

In view of the mechanical strength of the cured product, the self-adhesive dental composite resin preferably comprises a polymerization accelerator (e), along with the water-soluble photopolymerization initiator (c-1a), water-insoluble photopolymerization initiator (c-1b), and chemical polymerization initiator (c-2).

Examples of the polymerization accelerator (e) that can be used in the present invention include amines, sulfinic

acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds.

The amines used as polymerization accelerator (e) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the self-adhesive dental composite resin, preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino) benzophenone, and butyl 4-(N,N-dimethylamino)benzoate. In view of the ability to impart excellent curability to the self-adhesive dental composite resin, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, barbituric acid derivatives, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (e) may be contained alone, or two or more thereof may be contained in combination. The content of the polymerization accelerator (e) used in the present invention is not particularly limited. However, in view of the curability and other properties of the self-adhesive dental composite resin obtained, the content of polymerization accelerator (e) is preferably 0.001 to 30 parts by mass, more preferably 0.01 to 10 parts by mass, even more preferably 0.1 to 5 parts by mass relative to total 100 parts by mass of monomers in the self-adhesive dental composite resin. When the content of polymerization accelerator (e) is at or above these lower limits, it is easier to provide a sufficient bond strength by allowing polymerization to sufficiently proceed. In this respect, the content of polymerization accelerator (e) is more preferably 0.05 parts or more by mass. When the content of polymerization accelerator (e) is at or below the foregoing upper limits, it is easier to provide sufficient adhesive properties, and to reduce precipitation of the polymerization accelerator (e) itself from the self-adhesive dental composite resin. In this respect, the content of polymerization accelerator (e) is more preferably 20 parts or less by mass.

<Fluorine-Ion Releasing Substance>

A self-adhesive dental composite resin of the present invention may additionally comprise a fluorine-ion releasing substance. By containing a fluorine-ion releasing substance, the self-adhesive dental composite resin produced can impart acid resistance to tooth structure. Examples of the fluorine-ion releasing substance include fluorine-ion releasing polymers such as copolymers of methyl methacrylate and methacrylic acid fluoride; and metal fluorides such as sodium fluoride, potassium fluoride, sodium monofluorophosphate, lithium fluoride, and ytterbium fluoride. The fluorine-ion releasing substance may be incorporated alone, or two or more thereof may be incorporated in combination.

A self-adhesive dental composite resin of the present invention may also comprise a known additive, provided that such additives do not cause a performance drop.

Examples of additives that may be contained in the self-adhesive dental composite resin include polymerization inhibitors, antioxidants, colorants (pigments, dyes), ultraviolet absorbers, fluorescent agents, solvents such as organic solvents, thickeners, and chain transfer agents (such as $\alpha$-alkylstyrene compounds). The additives may be used alone, or two or more thereof may be used in combination.

In certain embodiments, the content of the solvent (for example, water, organic solvent) in the self-adhesive dental composite resin is preferably less than 1 mass%, more preferably less than 0.1 mass%, even more preferably less than 0.01 mass% in the total mass of the self-adhesive dental composite resin.

Another embodiment is, for example, a self-adhesive dental composite resin that does not comprise an $\alpha$-alkylstyrene compound.

Yet another embodiment is, for example, a self-adhesive dental composite resin that does not comprise a chain transfer agent.

In view of adjusting storage stability and curability, a self-adhesive dental composite resin of the present invention preferably comprises a polymerization inhibitor. Examples of the polymerization inhibitor include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. These may be used alone, or two or more thereof may be used in combination.

The content of polymerization inhibitors is preferably 0.001 to 1.0 parts by mass with respect to total 100 parts by mass of monomers in the self-adhesive dental composite resin.

In view of photostability against ambient light from light sources such as fluorescent lamps and LEDs, and reducing discoloration of the cured product, a self-adhesive dental composite resin of the present invention preferably comprises a ultraviolet absorber. Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(3-tert-butyl-2-hydroxy-5-methylphenyl)-5-chloro-2H-benzotriazole (Tinuvin 326); and benzoimidazole compounds. Preferred is Tinuvin 326. These may be used alone, or two or more thereof may be used in combination.

From an aesthetic perspective to reproduce a shade close to that of tooth structure, a self-adhesive dental composite resin of the present invention preferably comprises a fluorescent agent. While known fluorescent agents can be used without limitations, phthalic acid ester fluorescent agents are preferred.

Specific examples of phthalic acid ester fluorescent agents include dimethyl 2,5-dihydroxyterephthalate, diethyl 2,5-dihydroxyterephthalate, dimethylaminoterephthalate, and diethylaminoterephthalate. More preferred are phthalic acid ester fluorescent agent substituted with hydroxyl groups, such as diethyl 2,5-dihydroxyterephthalate. The fluorescent agent may be used alone, or two or more thereof may be used in combination.

The content of fluorescent agent relative to 100 parts by mass of the monomer components is not particularly limited. However, a certain quantity of fluorescent agent is necessary to ensure fluorescence. If the quantity is too high, it becomes difficult to maintain photostability. For a good balance between photostability and fluorescence, the content of fluorescent agent is preferably 0.005 to 0.5 parts by mass, more preferably 0.01 to 0.1 parts by mass.

In order to reproduce a shade close to that of tooth structure, a self-adhesive dental composite resin of the present invention preferably comprises a colorant. The type of colorant is not particularly limited, and any inorganic pigments and/or organic pigments can be used without restrictions, according the shade desired for the dental curable composition. The colorant may be used alone, or two or more thereof may be used in combination.

The colorant is a component added to the self-adhesive dental composite resin in trace amounts. For each type of colorant, the colorant content is less than 1.0 mass% in the total mass of the self-adhesive dental composite resin.

The colorant may be one with a refractive index of more than 2.00. The colorant may have a refractive index of 2.05 or more, or 2.10 or more.

The shape of colorant is not particularly limited, and the colorant may have any particle shape, including spherical, needle-shape, plate-shape, crushed shape, and scale-like, without any restrictions.

Examples of the inorganic pigments include:

chromates such as chrome yellow, zinc yellow, and barium yellow;
ferrocyanides such as iron blue;
sulfides such as silver vermilion, cadmium yellow, zinc sulfide, and cadmium red;
sulfates such as barium sulfate, zinc sulfate, and strontium sulfate;
oxides such as zinc white, antimony white, titanium white, red iron oxide, iron black, and chromium oxide;
hydroxides such as aluminum hydroxide;
silicates such as calcium silicate, and ultramarine; and
carbons such as carbon black, and graphite.

Examples of the organic pigments include

nitroso pigments such as naphthol green B, and naphthol green Y;
nitro pigments such as naphthol yellow S, and xylene fast yellow 2G;
insoluble azo pigments such as toluidine red, brilliant fast scarlet, Hansa yellow, and pigment yellow 14;
poorly soluble azo pigments such as lithol red, lake red C, and lake red D;
soluble azo pigments such as brilliant carmine 6B, toluidine red F5R, pigment scarlet 3B, and Bordeaux 10B;
phthalocyanine pigments such as phthalocyanine blue, phthalocyanine green, and sky blue;
basic dye pigments such as rhodamine lake, malachite green lake, and methyl violet lake; and
acidic dye pigments such as peacock blue lake, eosin lake, quinoline yellow lake, and aluminum lake.

Among these colorants, inorganic pigments such as titanium white, red iron oxide, iron black, and yellow ferrous oxide are preferred over the organic pigments due to their superior heat resistance and lightfastness.

The content of the colorant in a self-adhesive dental composite resin of the present invention is not particularly limited, as long as it falls within the effective range of the present invention. However, in view of aesthetics, the colorant content is preferably 0.0005 parts or more by mass, more preferably 0.002 parts or more by mass, even more preferably 0.004 parts or more by mass, particularly preferably 0.006 parts or more by mass relative to 100 parts by mass of the monomer components. With these lower limits, the filled area created by filling the self-adhesive dental composite resin into cavities can be prevented from appearing dark and dull.

The colorant content is preferably 2.0 parts or less by mass, more preferably 1.0 part or less by mass, even more preferably 0.5 parts or less by mass, particularly preferably 0.3 parts or less by mass relative to 100 parts by mass of the monomer components. With these upper limits, the filled area can effectively reflect the shade of natural teeth at the floor of cavities.

The colorant content is preferably 0.00001 mass% or more, more preferably 0.0001 mass% or more, even more preferably 0.0005 mass% or more, particularly preferably 0.001 mass% or more in the total mass of the self-adhesive dental composite resin. The colorant content is preferably 0.5 mass% or less, more preferably 0.3 mass% or less, even more preferably 0.1 mass% or less, particularly preferably 0.07 mass% or less.

Examples of the preferred composition ratios of the self-adhesive dental composite resin are as follows.

The self-adhesive dental composite resin comprises preferably 1 to 40 parts by mass of monomer (a) having an acidic group, and 60 to 99 parts by mass of monomer (b) having no acidic group in 100 parts by mass of the monomer components when the total amount of monomers is 100 parts by mass, and 0.05 to 10 parts by mass of photopolymerization initiator (c-1), 100 to 900 parts by mass of filler (d), and 0.001 to 30 parts by mass of polymerization accelerator (e) relative to total 100 parts by mass of the monomers, more preferably 2.5 to 35 parts by mass of monomer (a) having an acidic group, and 65 to 97.5 parts by mass of monomer (b) having no acidic group in total 100 parts by mass of the monomers, and 0.1 to 5 parts by mass of photopolymerization initiator (c-1), 120 to 560 parts by mass of filler (d), and 0.01 to 10 parts by mass of polymerization accelerator (e) relative to total 100 parts by mass of the monomers, even more preferably 5 to 30 parts by mass of monomer (a) having an acidic group, and 70 to 95 parts by mass of monomer (b) having no acidic group in total 100 parts by mass of the monomers, and 0.15 to 2.5 parts by mass of photopolymerization initiator (c-1), 150 to 400 parts by mass of filler (d), and 0.1 to 5 parts by mass of polymerization accelerator (e) relative to total 100 parts by mass of the monomers.

A self-adhesive dental composite resin comprising a monomer (a) having an acidic group, a monomer (b) having no acidic group, a polymerization initiator (c), and a filler (d) along with optional components can be produced with ease using methods known to a person skilled in the art.

A self-adhesive dental composite resin of the present invention may be combined with materials such as dental etchants, dental primers, and dental bonding materials. In view of bond strength to tooth structure, and cavity sealing properties (particularly, cavity sealing properties in wedge-shaped defect cavities) and the durability of cavity sealing properties, it is preferable to combine dental primers and dental bonding materials, more preferably dental bonding materials. The dental primers and dental bonding materials may or may not be polymerized alone. However, in view of cavity sealing properties (particularly, cavity sealing properties in wedge-shaped defect cavities) and the durability of cavity sealing properties, it is more preferable to polymerize these by photopolymerization or chemical polymerization. The dental primers and dental bonding materials may be used individually or in combination. In view of cavity sealing properties (particularly, cavity sealing properties in wedge-shaped defect cavities) and the durability of cavity sealing properties, it is preferable to employ a procedure that applies dental bonding materials after the application of dental primers.

<Specific Method of Application and Procedure>

The self-adhesive dental composite resin is applied to the surface of tooth structure in need of application.

For self-adhesive dental composite resins that contain a photopolymerization initiator, the self-adhesive composite resin is cured by exposing it to light with a dental photoirradiator after filling and smoothing the surface. The self-adhesive dental composite resin may be left to cure to completion when it contains a chemical polymerization initiator. This is followed by optional shape modification and polishing of the surface.

When the self-adhesive dental composite resin is used with materials such as dental etchants, dental primers, and dental bonding materials, these are used before applying the self-adhesive dental composite resin

EXAMPLES

The following describes the present invention in detail using Examples and Comparative Examples. However, the present invention is not limited to the following Examples. In the following, "part(s)" means "part(s) by mass", unless otherwise specifically stated.

The components of the self-adhesive dental composite resins of Examples and Comparative Examples are presented below, along with the abbreviations.

[Monomer (a) having an acidic group]

MDP: 10-methacryloyloxydecyl dihydrogen phosphate
GPDM: 1,3-dimethacryloyloxypropyl-2-dihydrogen phosphate
4-META: 4-methacryloyloxyethyl phthalic acid anhydride

[Monomer (b) having no acidic group]

D-2.6E: 2,2-bis(4-methacryloyloxypolyethoxyphenyl)propane (with an average of 2.6 moles of ethoxy group added)
Bis-GMA: 2,2-bis[4-(2-hydroxy-3-methacryloyloxypropoxy)phenyl]propane
3G: triethylene glycol dimethacrylate
DD: 1,10-decanediol dimethacrylate
MAEA: N-methacryloyloxyethylacrylamide
DEAA: N,N-diethylacrylamide
HEMA: 2-hydroxyethyl methacrylate
THF-MA: tetrahydrofurfuryl methacrylate
BEMA: benzyl methacrylate
POB-MA: phenoxybenzyl methacrylate
PEMA: 2-phenoxyethyl methacrylate
UN7700: urethane acrylate (manufactured by Negami Chemical Industrial Co., Ltd., weight-average molecular weight (Mw): 15,000 to 25,000, glass transition temperature (Tg): -41°C, polyester skeleton, the number of polymerizable groups: 2, weight-average molecular weight per acryl group: 7,500 to 12,500)
UN7600: urethane acrylate (manufactured by Negami Chemical Industrial Co., Ltd., weight-average molecular weight (Mw): 11,500, glass transition temperature (Tg): -42°C and 44.6°C, the number of polymerizable groups: 2, weight-average molecular weight per polymerizable group: 5,750)
Note that the weight-average molecular weight (Mw) refers to a weight-average molecular weight measured by gel permeation chromatography (GPC) in terms of polystyrene.

[Photopolymerization initiator (c-1)]

· Water-soluble photopolymerization initiator (c-1a)

Li-TPO: lithium phenyl(2,4,6-trimethylbenzoyl)phosphinate
BAPO-ONa: sodium bis(2,4,6-trimethylbenzoyl)phosphinate

· Water-insoluble photopolymerization initiator (c-1b)
CQ: camphorquinone

[Filler (d)]

The ratio of the constituents in each filler was determined by calculation from the mixing quantities or measured through calculation using a fluorescence X-ray analyzer (ZSX Primus-μ manufactured by Rigaku Corporation) (n = 3). The average particle diameters of the commercially available products are based on the values provided by the manufacturers. Unless specified otherwise, a scanning electron microscope (SU3500, manufactured by Hitachi, Ltd.) can be used to capture electron microscope images, and the particles size of randomly selected 200 particles can be measured to determine the average particle diameter. For non-spherical particles, the particle diameter is defined as the arithmetic mean value of the longest and shortest lengths of the particle.

Filler 1: Silane-Treated Silica

A three-neck flask was charged with 100 g of Ar 130 (manufactured by Nippon Aerosil Co., Ltd., hydrophilic fumed silica, ultrafine particulate silica Aerosil® 130, average particle diameter: 16 nm, refractive index: 1.46), 30 g of γ-methacryloyloxypropyltrimethoxysilane, and 200 mL of a 0.3 mass% acetic acid aqueous solution. These were stirred for 2 hours at room temperature under ultrasonic dispersion. After freeze drying to remove water, the mixture was heat-treated at 90°C for 3 hours to obtain filler 1.

Filler 2: Silane-Treated Silica-Coated Ytterbium Fluoride

Surface-treated silica-coated ytterbium fluoride (SG-YBF100WSCMP10, average particle diameter of primary particles: 110 nm, average particle diameter of secondary particles: 1.2 μm, refractive index: 1.53, manufactured by Sukgyung AT)

Filler 3: Silane-Treated Silica Stone Powder (Quartz)

A silica stone powder (manufactured by Nitchitsu Co., Ltd. under the trade name Hi-Silica, refractive index: 1.55) was pulverized with a dry ball mill (10-mm diameter alumina balls) to obtain a pulverized silica stone powder. The pulverized silica stone powder had an average particle diameter of 2.2 μm as measured by volume with a laser diffraction particle size distribution analyzer (Model SALD-2300, manufactured by Shimadzu Corporation). Subsequently, 100 g of this pulverized silica stone powder was charged into a three-neck flask along with 4 g of γ-methacryloyloxypropyltrimethoxysilane, and 200 mL of an acetic acid aqueous solution. These were stirred for 2 hours at room temperature under ultrasonic dispersion. After freeze drying to remove water, the mixture was heat-treated at 90°C for 3 hours to obtain filler 3.

Filler 4: Silane-Treated Barium Glass Powder (Barium Glass)

A barium glass (manufactured by Esstech, product code E-3000, refractive index: 1.56) was pulverized with a dry ball mill (10-mm diameter alumina balls) to obtain a barium glass powder. The barium glass powder had an average particle diameter of 2.4 μm as measured by volume with a laser diffraction particle size distribution analyzer (Model SALD-2300, manufactured by Shimadzu Corporation). Subsequently, 100 g of this barium glass powder was charged into a three-neck flask along with 4 g of γ-methacryloyloxypropyltrimethoxysilane, and 200 mL of an acetic acid aqueous solution. These were stirred for 2 hours at room temperature under ultrasonic dispersion. After freeze drying to remove water, the mixture was heat-treated at 90°C for 3 hours to obtain filler 4.

Filler 5: Silane-Treated Silica Stone Powder (Quartz)

A silica stone powder (manufactured by Nitchitsu Co., Ltd. under the trade name Hi-Silica, refractive index: 1.55) was pulverized with a dry ball mill (10-mm diameter alumina balls). After further pulverization with a wet bead mill (1-mm diameter zirconia beads), the pulverized silica stone powder had an average particle diameter of 0.5 μm as measured by volume with a laser diffraction particle size distribution analyzer (Model SALD-2300, manufactured by Shimadzu Corporation). Subsequently, 100 g of this pulverized silica stone powder was charged into a three-neck flask along with 10 g of γ-methacryloyloxypropyltrimethoxysilane, and 200 mL of a 0.5% acetic acid aqueous solution. These were stirred for 2 hours at room temperature under ultrasonic dispersion. After freeze drying to remove water, the mixture was heat-treated at 90°C for 3 hours to obtain filler 5.

Filler 6: Production of Silane-Treated Aggregate Inorganic Particles

A three-neck flask was charged with 100 parts by mass of Silica Microbead P-500 (average particle diameter of primary particles: 12 nm, average particle diameter of secondary particles: 2.0 μm, refractive index: 1.46; manufactured by JGC C & C), 20 parts by mass of 3-methacryloyloxypropyltrimethoxysilane, and 200 mL of a 0.5% acetic acid aqueous solution. These were stirred for 2 hours at room temperature under ultrasonic dispersion. After freeze drying to remove water, the mixture was heat-treated at 90°C for 3 hours to obtain filler 6. The filler 6 had an average particle diameter of 2.0 μm as measured by volume with a laser diffraction particle size distribution analyzer (Model SALD-2300, manufactured by Shimadzu Corporation).

Filler 7: Surface-Treated Organic-Inorganic Composite Filler

A mixture of 70 parts by mass of Bis-GMA, 30 parts by mass of 3G, and 0.5 parts by mass of benzoyl peroxide was

uniformly dissolved to obtain a monomer-containing composition. Separately, a colloidal silica powder with an average particle diameter of 0.04 μm (Aerosil® OX50, manufactured by Nippon Aerosil Co., Ltd.) was surface-treated with γ-methacryloyloxypropyltrimethoxysilane using an ordinary method. Subsequently, 100 parts by mass of the surface-treated colloidal silica and 100 parts by mass of the polymerizable monomer were kneaded into a paste-form composition. The composition was heated at 130°C for 3 hours under reduced pressure to polymerize, and the resulting cured product was pulverized with a ball mill to obtain an organic-inorganic composite filler with no surface treatment. One-hundred parts by mass of this surface-untreated organic-inorganic composite filler was then subjected to surface treatment with 3 parts by mass of γ-methacryloyloxypropyltrimethoxysilane to obtain filler 7. The filler 7 had an average particle diameter of 11 μm as measured by volume with a laser diffraction particle size distribution analyzer (Model SALD-2300, manufactured by Shimadzu Corporation). The refractive index was 1.50.

Filler 8: hydrophobic fumed silica manufactured by Nippon Aerosil Co., Ltd., ultrafine particulate silica Aerosil® R972, average particle diameter: 16 nm (silica), refractive index: 1.46

Filler 9: spherical crosslinked polystyrene particle SBX-4 (TECHPOLYMER® SBX-4, manufactured by Sekisui Kasei Co., Ltd., average particle diameter: 4 μm, refractive index: 1.59)

Filler 10: Aerosil® 380, particulate silica AEROSIL® 380 manufactured by Nippon Aerosil Co., Ltd. (hydrophilic fumed silica, average particle diameter: 7 nm, refractive index: 1.46)

Table 1 summarizes the properties of the fillers 1 to 10.

[Table 1]

| Filler | Classification | Average particle diameter (μm) | Refractive index | Shape | Ratio of constituents (mass%) | Constituents |
|---|---|---|---|---|---|---|
| Filler 1 | Inorganic filler | 0.016 | 1.46 | Irregular | $SiO_2$:100 | $SiO_2$ |
| Filler 2 | Inorganic filler | 1.2 | 1.53 | Irregular | $YbF_3$:95, $SiO_2$:5 | $SiO_2$-coated $YbF_3$ |
| Filler 3 | Inorganic filler | 2.2 | 1.55 | Irregular | $SiO_2$:100 | $SiO_2$ |
| Filler 4 | Inorganic filler | 2.4 | 1.56 | Irregular | $SiO_2$,:50 BaO:30, $Al_2O_3$:10, $B_2O_3$:10 | $SiO_2$, BaO, $Al_2O_3$, $B_2O_3$ |
| Filler 5 | Inorganic filler | 0.5 | 1.55 | Irregular | $SiO_2$:100 | $SiO_2$ |
| Filler 6 | Inorganic filler | 2.0 | 1.46 | Spherical | $SiO_2$:100 | $SiO_2$ |
| Filler 7 | Organic-inorganic composite filler | 11 | 1.50 | Irregular | $SiO_2$:50,Organic material:50 | Organic material, $SiO_2$ |
| Filler 8 | Inorganic filler | 0.016 | 1.46 | Irregular | $SiO_2$:100 | $SiO_2$ |
| Filler 9 | Organic filler | 4 | 1.59 | Spherical | Organic material:100 | Organic material |
| Filler 10 | Inorganic filler | 0.007 | 1.46 | Irregular | $SiO_2$:100 | $SiO_2$ |

[Polymerization accelerator (e)]

DABE: ethyl 4-(N,N-dimethylamino)benzoate

[Others]

TN326: 2-(3-tert-butyl-2-hydroxy-5-methylphenyl)-5-chloro-2H-benzotriazole (manufactured by BASF Japan under the trade name Tinuvin 326; ultraviolet absorber)
LBL: diethyl 2,5-dihydroxyterephthalate (fluorescent agent)
BHT: 3,5-di-t-butyl-4-hydroxytoluene (polymerization inhibitor)

[Colorant]

$TiO_2$: titanium oxide (refractive index: 2.50 or more, pigment)
Iron oxide (yellow) (refractive index: 2.10, pigment)
Iron oxide (red) (refractive index: 3.01, pigment)
Iron oxide (black) (refractive index: 2.42, pigment)

[Preparation of self-adhesive dental composite resin]

The raw materials listed in Tables 2 to 4 were mixed and kneaded at ordinary temperature (23°C) in a dark environment, and the resulting uniform material was subjected to vacuum defoaming to prepare a paste-form self-adhesive dental composite resin.
The self-adhesive dental composite resin was filled into a stainless-steel mold (10 mm in diameter × 1 mm in thickness). With glass slides pressed against the mold on the upper and lower sides, the composite resin was cured by exposing it to light from both sides, 45 seconds each, through the contacting glass slides, using a dental visible-light irradiator (α-Light V, manufactured by J. Morita Corp.). This resulted in a plate-shaped cured product.
Samples of this cured product were evaluated for lightness (L*/w) and chromaticity (a*/w, b*/w) against a white background using a spectrophotometer (SE6000 manufactured by Nippon Denshoku Industries Co., Ltd., illuminant/field: D65/2 degrees, measurement aperture: 6 mm in diameter).
Specifically, lightness (L*/w) and chromaticity (a*/w, b*/w) were measured according to the L*a*b* color system with a standard white board placed behind the plate-shaped cured product (n = 1).
Subsequently, the lightness (L*/w) and chromaticity (a*/w, b*/w) of shade A2 of a shade guide (VITA Classical Shade Guide manufactured by VITA under this trade name) were measured against a white background, using the same spectrophotometer under the same conditions.
The lightness and chromaticity of the cured product against a white background were then evaluated in terms of a color difference ΔE*w from the lightness and chromaticity of the A2 shade against a white background, using the following formula (n = 1).

$$\Delta E^{*}w = ((L_1^{*}/w - L_0^{*}/w)^2 + (a_1^{*}/w - a_0^{*}/w)^2 + (b_1^{*}/w - b_0^{*}/w)^2)^{1/2}$$

In the formula, $L_1^{*}/w$, $a_1^{*}/w$, and $b_1^{*}/w$ represent the lightness (L value) and chromaticity (a value and b value) of the cured product of the self-adhesive dental composite resin against a white background. $L_0^{*}/w$, $a_0^{*}/w$, and $b_0^{*}/w$ represent the lightness (L value) and chromaticity (a value and b value) of the A2 shade against a white background.
In Test Examples 1 to 8, with the exception of Comparative Examples 1 to 4, pigments (colorants) were added in suitable amounts to ensure a color difference ΔE* of less than 6.0 from the A2 shade. Using these self-adhesive dental composite resins, various properties were evaluated. In the tables, samples containing pigments are indicated by "Added" in the pigment row. The evaluation results are presented in Tables 2 to 4.

Test Example 1: Polymerization Shrinkage Stress

A 5.0 mm thick glass plate, treated by sandblasting with 50 μm alumina powder, was coated with a dental ceramic adhesive material (manufactured by Kuraray Noritake Dental Inc. under the trade name Clearfil® Ceramic Primer Plus) serving as a pretreatment material, and dried by blowing air.
A stainless-steel washer (inner diameter 5.3 mm × 0.8 mm thickness), separately prepared and coated with a release agent, had a dental adhesive (Clearfil® Mega Bond® 2 Bond, manufactured by Kuraray Noritake Dental Inc. under this trade name) applied to it without any excess. With the washer pressed onto the glass plate, the washer was secured to

the glass plate by applying light for 10 seconds in standard mode from the side of the glass plate not in contact with the washer, using a dental visible-light irradiator (PenCure 2000, manufactured by J. Morita Corp.).

Subsequently, in order to secure the glass plate to the self-adhesive dental composite resin, a Clearfil® Mega Bond® 2 Bond was applied inside the washer, and light was applied for 10 seconds in standard mode using a dental visible-light irradiator (PenCure 2000, manufactured by J. Morita Corp.). Following this, the self-adhesive dental composite resin was filled into the washer in paste form.

A Clearfil® Mega Bond® 2 Bond was applied to a separately prepared stainless-steel fixture (5 mm in diameter) treated by sandblasting. From the applied side, the bond was exposed to light for 10 seconds in standard mode, using a dental visible-light irradiator (PenCure 2000, manufactured by J. Morita Corp.). After this exposure of the bond to light, the self-adhesive composite resin paste was placed between the stainless-steel fixture and the glass plate inside the washer, and any excess paste was removed.

In order to cure the self-adhesive dental composite resin, the paste filling the washer was exposed to light for 10 seconds in standard mode from the glass plate side, using a dental LED photoirradiator (manufactured by J. Morita Corp. under the trade name PenCure 2000). The stress exerted after 3 minutes from exposure to light was then measured as polymerization shrinkage stress using a universal testing machine (Autograph AG-I 100kN, Shimadzu Corporation) (n = 3), and the average value was calculated.

In view of cavity sealing properties (particularly, cavity sealing properties in wedge-shaped defect cavities) and the durability of cavity sealing properties, the polymerization shrinkage stress needs to be less than 10.0 MPa, preferably less than 9.5 MPa, more preferably less than 9.0 MPa, even more preferably less than 8.5 MPa.

Test Example 2: Degree of Light Diffusion (D) of Cured Product

The prepared paste-form self-adhesive dental composite resin was filled into a Teflon® mold (30 mm in diameter × 0.5 mm in thickness). With glass slides pressed against the mold on the upper and lower sides, the composite resin was cured by exposing it to light from both sides, 45 seconds each, through the contacting glass slides, using a dental visible-light irradiator (α-Light V, manufactured by J. Morita Corp.). This resulted in a plate-shaped cured product.

The cured product was used as a sample plate (30 mm in diameter × 0.5 mm in thickness) to measure the luminous intensity distribution of transmitted light, using a three-dimensional goniometer (GP-200 manufactured by Murakami Color Research Laboratory Co., Ltd., with a halogen light source and a color temperature of approximately 6774 K) (n = 3). The degree of light diffusion D was calculated according to the following formula [I], and the average value of the measured degrees of light diffusion D was calculated.

$$D = (I_{20}/\cos 20° + I_{70}/\cos 70°)/(2I_0) \qquad [I]$$

where I represents the luminous intensity of light transmitted through a sample plate made of the self-adhesive dental composite resin after curing, and $I_0$, $I_{20}$, and $I_{70}$ represent the luminous intensity values (intensity of light) at 0-degree, 20-degree, and 70-degree angles, respectively, relative to the direction perpendicular to the sample plate (the direction of light incidence).

The cured product needs to have a degree of light diffusion D of 0.10 or more, preferably 0.12 or more, more preferably 0.15 or more, even more preferably 0.18 or more, particularly preferably 0.20 or more. In view of enhancing the transparency and shade conformity of the cured product, the degree of light diffusion D is less than 3.00, preferably 2.80 or less, more preferably 2.50 or less, even more preferably 2.00 or less. In view of even superior shade conformity to both enamel and dentin, the degree of light diffusion D is particularly preferably 1.00 or less.

Test Example 3: Water Absorption and Dissolution

The cured product of the self-adhesive dental composite resin was evaluated for water absorption and dissolution by water absorption and dissolution tests, in compliance with ISO 4049:2019. The detailed procedures are as follows.

The prepared paste-form self-adhesive dental composite resin was filled into a SUS mold (15 mm in diameter, 1.0 mm in thickness). The paste was then pressed with glass slides after placing polyester films on the top and bottom surfaces (15 mm in diameter) of the paste. Under the pressure of the glass slides, the paste was exposed to light through the glass slides, using a dental visible-light irradiator (PenCure 2000, manufactured by J. Morita Corp.). Here, light was applied for 10 seconds from both sides of the paste in standard mode, at seven different points on each side (a total of 70 seconds of light exposure per side). This process cured the paste and produced a cured product.

The cured product was placed as a sample in a desiccator maintained at 37°C. The desiccation process was repeated until the sample had a mass decrease of 0.1 mg or less within 24 hours. Once the sample reached a constant

mass (where no further mass change occurs despite continuous drying in the desiccator), the final mass was recorded as $m_1$.

The sample was also measured for diameter and thickness to determine its volume (V), using a micrometer. The sample was then immersed in distilled water (30 mL) in a container, and left in this state for 7 days inside a thermostatic chamber set to 37°C. After this period, the sample was taken out of distilled water, and the surface water was removed. The resulting mass of the sample was recorded as $m_2$.

The sample was then placed in a desiccator maintained at 37°C. The desiccation process was repeated until the sample had a mass decrease of 0.1 mg or less within 24 hours. Once the sample reached a constant mass, the final mass was recorded as $m_3$. A total of five samples were prepared for each Example and Comparative Example, and the average value was calculated (n = 5).

The water absorption was determined using the following formula.

$$W_{sp}\ (\mu g/mm^3) = (m_2 - m_3)\ (\mu g) / V\ (mm^3)$$

$m_2$: mass of the sample after 7 days of immersion in water (μg)
$m_3$: mass of the sample at its constant weight (μg)
V: volume of the sample ($mm^3$)

The dissolution was determined using the following formula.

$$W_{sl}\ (\mu g/mm^3) = (m_1 - m_3)\ (\mu g) / V\ (mm^3)$$

$m_1$: mass of the sample adjusted before immersion in water (μg)
$m_3$: mass of the sample at its constant weight (μg)
V: volume of the sample ($mm^3$)

In view of the durability of the cured product, smaller values are preferred for water absorption, preferably less than 40 μg/$mm^3$, more preferably 30 μg/$mm^3$ or less, even more preferably 20 μg/$mm^3$ or less, particularly preferably 15 μg/$mm^3$ or less.

In view of the durability of the cured product, smaller values are preferred for dissolution, preferably 7.5 μg/$mm^3$ or less, more preferably 5.0 μg/$mm^3$ or less, even more preferably 3.5 μg/$mm^3$ or less, particularly preferably 2.0 μg/$mm^3$ or less.

Test Example 4: Shear Bond Strength (Enamel, Dentin)

The test was conducted in compliance with ISO29022:2013, specifically as follows.

The labial surfaces of bovine teeth were polished with #80 silicon carbide paper (manufactured by Nihon Kenshi Co., Ltd.) under running water to obtain samples with exposed flat enamel surfaces and samples with exposed flat dentin surfaces.

First, samples with exposed flat enamel surfaces were used to measure shear bond strength to enamel, as follows.

A mold with 15 holes (15-hole mold, manufactured by Ultradent Products Inc., 35 mm in diameter × 25 mm in height) was separately prepared and a tape was attached to the bottom of the mold. The bovine tooth sample was then secured onto the tape.

Thereafter, a resin for dental impression trays (Tray Resin II manufactured by Shofu Inc. under this trade name) was filled into the mold, and left to stand for about 30 minutes to cure. This resulted in a composite of bovine tooth and cured resin. This composite was then removed from the mold to obtain a sample.

The composite had the bovine tooth exposed at the top surface of the cured resin. The top surface of the sample was polished under running water to a size large enough to provide a bonding surface (a diameter of 2.38 mm or more), using a #600 silicon carbide paper (manufactured by Nihon Kenshi Co., Ltd.), and the bonding surface was ultrasonically washed with water for 5 minutes.

Subsequently, a separately prepared CR filling mold (Bonding Mold Insert, manufactured by Ultradent Products Inc.; Ø = 2.38 mm) was installed on a dedicated instrument (Bonding Clamp, manufactured by Ultradent Products Inc.). The sample was secured by lowering the CR filling mold, allowing the CR filling mold installed on the dedicated instrument to contact the bonding surface of the sample.

Thereafter, the prepared self-adhesive dental composite resin was filled into the hole of the CR filling mold to form

a thin layer of no greater than 1 mm. After filling another portion into the CR filling mold (to about 2/3 of the mold, or about 2 mm thick), the self-adhesive dental composite resin was left to stand for 10 seconds, and irradiated with light for 10 seconds to cure, using a dental LED photoirradiator (manufactured by Ultradent Products Inc. under the trade name VALO). The sample was removed from the CR filling mold, and used as an adhesion test sample. A total of ten adhesion test samples were prepared.

The adhesion test sample was immersed in distilled water inside a container, and was left to stand for 24 hours in this state in a thermostatic chamber with the chamber temperature set to 37°C. The sample was immediately measured for its shear bond strength to enamel after being taken out of distilled water. The average values of the measurements are indicated by "Initial" in the Tables.

For the measurement of bond strength (shear bond strength), the adhesion test sample was installed on a dedicated holder (Test Base Clamp, manufactured by Ultradent Products Inc.), and the bond strength was measured using a dedicated jig (Crosshead Assembly, manufactured by Ultradent Products Inc.) and a universal testing machine (manufactured by Shimadzu Corporation) with the crosshead speed set at 1 mm/min. The average values are presented in the Tables (n = 10).

The shear bond strength to dentin was also measured in the same manner. The average values are presented in the Tables (n = 10).

In view of cavity sealing properties (particularly, cavity sealing properties in wedge-shaped defect cavities) and the durability of cavity sealing properties, larger values are preferred for shear bond strength to enamel and dentin. The shear bond strength to enamel and dentin is preferably 4.0 MPa or more, more preferably 5.0 MPa or more, even more preferably 6.0 MPa or more, particularly preferably 8.0 MPa or more.

Test Example 5: Flexural Properties (Flexural Modulus, Flexural Strength)

The flexural modulus and flexural strength were evaluated by conducting a flexure test in compliance with ISO 4049:2019, specifically as follows.

The prepared paste-form self-adhesive dental composite resin was filled into a SUS mold (2 mm in length × 25 mm in width × 2 mm in thickness), and pressed with glass slides from the top and bottom (over a 2 mm × 25 mm surface). Under the pressure of the glass slides, the paste was exposed to light through the glass slides, using a dental visible-light irradiator (PenCure 2000, manufactured by J. Morita Corp.). Here, light was applied for 10 seconds from both sides of the paste in standard mode, at five different points on each side (a total of 50 seconds of light exposure per side). This process cured the paste and produced a cured product. A total of ten cured products were prepared for each Example and Comparative Example.

Five of the samples were immersed in distilled water in a container, and left to stand in this state for 24 hours inside a thermostatic chamber that had been set to 37°C. The three-point flexural strength and flexural modulus were measured at a span length of 20 mm and a crosshead speed of 1 mm/min, using a universal testing machine (Autograph AG-I 100kN, manufactured by Shimadzu Corporation) (n = 5), and the average values were calculated. The average values of the measurements are indicated by "Initial" in the Tables.

The remaining five samples were immersed in distilled water in a container, and left to stand in this state for 7 days inside a thermostatic chamber that had been set to 70°C. The three-point flexural strength and flexural modulus were measured at a span length of 20 mm and a crosshead speed of 1 mm/min, using a universal testing machine (Autograph AG-I 100kN, manufactured by Shimadzu Corporation) (n = 5), and the average values were calculated. The average values of the measurements are indicated by "Enduring" in the Tables.

In view of the mechanical strength of the cured product, the flexural strength (initial) is preferably 80 MPa or more, more preferably 85 MPa or more, even more preferably 90 MPa or more, particularly preferably 95 MPa or more, most preferably 100 MPa or more.

Similarly, in view of mechanical strength, the flexural strength (enduring) is preferably 60 MPa or more, more preferably 65 MPa or more, even more preferably 70 MPa or more, particularly preferably 75 MPa or more, most preferably 80 MPa or more.

In view of mechanical strength, the flexural modulus (both initial and enduring) is preferably 2.0 GPa or more, more preferably 3.0 GPa or more, even more preferably 4.0 GPa or more. In view of reducing polymerization shrinkage stress with consideration given to cavity sealing properties (particularly, cavity sealing properties in wedge-shaped defect cavities) and the durability of cavity sealing properties, the flexural modulus is preferably less than 8.0 GPa, more preferably less than 7.0 GPa, even more preferably less than 6.0 GPa, particularly preferably 5.4 GPa.

Test Example 6: Cavity Sealing Properties

The labial surfaces of bovine teeth were polished with #80 silicon carbide paper (manufactured by Nihon Kenshi Co., Ltd.) under running water to obtain samples with an exposed flat enamel and dentin surface on the same bovine tooth.

On this flat surface, a cylindrical cavity (4 mm in diameter × 1.5 mm in depth) was created with an air turbine to mimic a wedge-shaped defect cavity, with enamel on one marginal side and dentin on the other side. After washing the cavity with water and drying it by blowing air, the self-adhesive dental composite resin was filled into the cavity, left to stand for 10 seconds, and exposed to light for 10 seconds with a dental LED photoirradiator (manufactured by Ultradent Products Inc. under the trade name VALO). The resulting cured product served as a sample.

The surface of the cured product was smoothed with an air turbine and polished with a micromotor. The sample was immersed in distilled water in a container, and left to stand in this state for 24 hours in a thermostatic chamber that had been set to 37°C. The sample after exposure was examined with an optical coherence tomography device (optical coherence tomography: OCT, IVS-2000, manufactured by Santec) to assess its initial cavity sealing properties, using the following criteria (n = 3). The results are indicated by "Initial" in the Tables.

<Evaluation Criteria>

Score 1: Samples with detachment occurring only on sidewalls of cavity
Score 2: Detachment occurring at the cavity floor in any of samples
Score 3: Detachment occurring on sidewalls and the floor of cavity in any of samples

Additionally, the samples evaluated for initial cavity sealing properties were subjected to 4,000 cycles of thermal cycling involving alternating immersion in 4°C cold water and 60°C hot water for 1 minute each. After this thermal cycling, the sample was examined again using OCT to assess the durability of cavity sealing properties, using the same criteria used for the evaluation of initial cavity sealing properties. The results are indicated by "Enduring" in the Tables.

Smaller values are preferred for cavity sealing properties. The preferred score is 1 or less for initial cavity sealing properties. For enduring cavity sealing properties, the preferred score is 2 or less, more preferably 1 or less.

Test Example 7: Shade conformity of Cured Product

The shade conformity of the self-adhesive dental composite resin was assessed by filling and restoring cavities formed in bovine teeth to mimic wedge-shaped defect cavities, and measuring the color of the restored area with a dental colorimeter.

Specifically, the labial surfaces of bovine teeth were polished with #80 silicon carbide paper (manufactured by Nihon Kenshi Co., Ltd.) under running water to obtain samples with an exposed flat enamel and dentin surface on the same bovine tooth.

On this flat surface, a cylindrical cavity (4 mm in diameter × 1.5 mm in depth) was created with an air turbine to mimic a wedge-shaped defect cavity, with enamel on one marginal side and dentin on the other side. The cavity was washed with water and dried by blowing air.

The self-adhesive dental composite resin was filled into the cavity, left to stand for 10 seconds, and exposed to light for 10 seconds with a dental LED photoirradiator (manufactured by Ultradent Products Inc. under the trade name VALO). This resulted in a sample with the cured product filling the cavity. The surface of the cured product was smoothed with a diamond bur (manufactured by Shofu Inc. under the trade name Shofu Diamond Point FG, Type: Superfine, Form Number: SF104R), and polished with an abrasive (manufactured by Shofu Inc. under the trade name CompoMaster CA, Form Number: 13S).

The sample with the cured product filling the cavity was captured with a dental colorimeter (manufactured by Olympus Corporation under the trade name Crystaleye CE100-DC/JP, equipped with a 7-band LED light source and analysis software Crystaleye). Here, the sample bovine teeth with the cured product of the self-adhesive dental composite resin filling the cavity were measured in the dark box (check box, top cover) attached to the dental colorimeter.

Using the analysis software, the sample cured product was measured for lightness ($L^*_1$) and chromaticity ($a^*_1$, $b^*_1$) at the enamel margin in the captured image.

The lightness ($L^*_0$) and chromaticity ($a^*_0$, $b^*_0$) were also measured for areas of bovine teeth outside the areas filled with the cured product in the captured image at the enamel margin. The color difference $\Delta E^*$ was then calculated using the results from both measurement locations, using the following formula. This served as an indicator of shade conformity (n = 1).

$$\Delta E^* = ((L^*_1 - L^*_0)^2 + (a^*_1 - a^*_0)^2 + (b^*_1 - b^*_0)^2)^{1/2}$$

In the formula, $L^*_1$, $a^*_1$, and $b^*_1$ represent the lightness (L value) and chromaticity (a value, b value) of the cured product of

the self-adhesive dental composite resin at the enamel margin. $L^*_0$, $a^*_0$, and $b^*_0$ represent the lightness (L value) and chromaticity (a value, b value) of areas of bovine teeth at the enamel margin outside the areas filled with the cured product.

The same measurements were conducted for dentin, using the method employed for enamel. The cured product filling the cavity was measured for lightness ($L^*_1$) and chromaticity ($a^*_1$, $b^*_1$) at the dentin margin in the captured image. The lightness ($L^*_0$) and chromaticity ($a^*_0$, $b^*_0$) were also measured for areas of bovine teeth outside the areas filled with the cured product in the captured image at the dentin margin. The color difference ΔE* was calculated using the formula above (n = 1).

The shade conformity was evaluated using the following criteria for both enamel and dentin sides. In view of shade conformity to tooth structure, smaller values are preferred for shade conformity.

<Evaluation Criteria>

Score 1: ΔE* = 5.0 or less
Score 2: ΔE* = more than 5.0 and 5.5 or less
Score 3: ΔE* = more than 5.5 and 6.0 or less
Score 4: ΔE* = more than 6.0

In view of shade conformity to tooth structure, smaller values are preferred for shade conformity. The color difference ΔE* is preferably 5.5 or less, more preferably 5.0 or less.

Test Example 8: Polymerization Shrinkage Rate

In order to measure the paste volume ($V_1$; unit: $mm^3$), the paste was filled in the glass cell (10.0 mm in inner diameter × 10 mm in depth) of a dry automatic densitometer (AccuPyc 1330, constant volume expansion method with nitrogen gas, manufactured by Shimadzu Corporation). This dry automatic densitometer has a structure where the sample chamber is connected to the expansion chamber via a valve. By filling nitrogen gas and manipulating the valve to open and close, the device can measure the sample volume based on the resulting pressure changes between the sample chamber and expansion chamber.

Subsequently, the paste was exposed to light for 10 seconds from above the glass cell, using a dental visible-light irradiator (PenCure 2000, manufactured by J. Morita Corp.) in standard mode. Immediately after, the volume of the paste was measured with the densitometer ($V_2$; unit: $mm^3$). The polymerization shrinkage rate was then determined according to the following formula (n = 3).

$$\text{Polymerization shrinkage rate (\%)} = (V_1 - V_2) / V_1 \times 100$$

In view of cavity sealing properties, smaller values are preferred for polymerization shrinkage rate. The polymerization shrinkage rate is preferably 3.5% or less, more preferably 3.3% or less, even more preferably 3.1% or less.

[Table 2]

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|
| Monomer (a) having acidic group | MDP | 5 | 5 | 5 | 5 | 5 | 5 | 5 | 5 | 5 | 5 | 5 | 5 | 5 |
| | GPDM | | | | | | | | | | | | | |
| | 4-META | | | | | | | | | | | | | |
| Monomer (b) having no acidic group | D-2.6E | 55 | 55 | 55 | 55 | 55 | 55 | 55 | 55 | 55 | 55 | 55 | 55 | 55 |
| | Bis-GMA | | | | | | | | | | | | | |
| | MAEA | 5 | 5 | 5 | 5 | 5 | 5 | 5 | 5 | 5 | 5 | 5 | 5 | 5 |
| | DD | | | | | | | | | | | | | |
| | 3G | | | | | | | | | | | | | |
| | THF-MA | 15 | 15 | 15 | 15 | 15 | 15 | 15 | 15 | 15 | 15 | 15 | 15 | 15 |
| | BEMA | | | | | | | | | | | | | |
| | POB-MA | | | | | | | | | | | | | |
| | PEMA | | | | | | | | | | | | | |
| | HEMA | | | | | | | | | | | | | |
| | DEAA | | | | | | | | | | | | | |
| | UN7600 | 20 | 20 | 20 | 20 | 20 | 20 | 20 | 20 | 20 | 20 | 20 | 20 | 20 |
| | UN7700 | | | | | | | | | | | | | |
| Polymerization initiator (c) | CQ | 0.2 | 0.2 | 0.2 | 0.2 | 0.2 | 0.2 | 0.2 | 0.2 | 0.2 | 0.2 | 0.1 | 0.3 | 0.2 |
| | Li-TPO | | | | | | | | | | | | | 0.3 |
| | BAPO-ONa | 0.3 | 0.3 | 0.3 | 0.3 | 0.3 | 0.3 | 0.3 | 0.3 | 0.3 | 0.3 | 0.3 | | |
| **Filler (d)** | Filler 1 | 5 | 5 | 5 | 5 | 5 | 5 | 5 | 5 | 5 | 5 | 5 | 5 | 5 |
| | Filler 2 | 40 | 40 | 40 | 40 | 80 | 40 | 40 | | | 40 | 40 | 40 | 40 |
| | Filler 3 | 150 | 190 | 220 | 140 | 150 | | | 170 | 170 | 150 | 150 | 150 | 150 |
| | Filler 4 | | | | | | 190 | | | | | | | |
| | Filler 5 | | | | | | | 120 | | | | | | |

(continued)

| Components (parts by mass) | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|
| | Filler 6 | | | | | | | | | 20 | | | | | |
| | Filler 7 | | | | | | | | | | 20 | | | | |
| | Filler 8 | | | | | | | | | | | | | | |
| | Filler 9 | | | | | | | | | | | | | | |
| | Filler 10 | | | | | | | | | | | | | | |
| Polymerization accelerator (e) | DABE | | 0.2 | 0.2 | 0.2 | 0.2 | 0.2 | 0.2 | 0.2 | 0.2 | 0.2 | 0.4 | 0.1 | 0.4 | 0.2 |
| Others | TN326 | | 0.2 | 0.2 | 0.2 | 0.2 | 0.2 | 0.2 | 0.2 | 0.2 | 0.2 | 0.2 | 0.2 | 0.2 | 0.2 |
| | LBL | | 0.02 | 0.02 | 0.02 | 0.02 | 0.02 | 0.02 | 0.02 | 0.02 | 0.02 | 0.02 | 0.02 | 0.02 | 0.02 |
| | BHT | | 0.1 | 0.1 | 0.1 | 0.1 | 0.1 | 0.1 | 0.1 | 0.1 | 0.1 | 0.1 | 0.1 | 0.1 | 0.1 |
| | Pigment | | Added | Added | Added | Added | Added | Added | Added | Added | Added | Added | Added | Added | Added |
| Polymerization shrinkage stress | MPa | | 7.5 | 8.2 | 8.6 | 7.2 | 8.4 | 8.4 | 9.0 | 7.6 | 7.7 | 9.6 | 6.4 | 7.7 | 7.7 |
| Degree of light diffusion D of cured product | - | | 0.3 | 0.4 | 0.3 | 0.2 | 0.4 | 0.4 | 0.3 | 0.3 | 0.3 | 0.3 | 0.3 | 0.3 | 0.3 |
| Water absorp-tion | μg/mm$^3$ | | 14 | 13 | 12 | 16 | 14 | 13 | 20 | 14 | 13 | 12 | 16 | 14 | 14 |
| Shear bond strength (Enamel) | MPa | Initial | 8.8 | 8.0 | 8.0 | 9 | 8.5 | 8.2 | 8.1 | 8.5 | 8.2 | 8.3 | 7.6 | 5.6 | 8.7 |
| Shear bond strength (Dentin) | MPa | Initial | 7,8 | 7.6 | 7.5 | 8.3 | 8 | 7.7 | 7.5 | 8.1 | 7.6 | 7.6 | 6.8 | 5 | 7.8 |
| Flexural strength | MPa | Initial | 88 | 93 | 105 | 82 | 90 | 90 | 86 | 87 | 85 | 103 | 83 | 87 | 87 |
| Flexural strength | MPa | Enduring | 68 | 80 | 84 | 63 | 71 | 74 | 65 | 66 | 66 | 83 | 60 | 69 | 70 |

(continued)

| Components (parts by mass) | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|
| Cavity sealing properties | - | Initial | 1 | 1 | 1 | 1 | 1 | 1 | 1 | 1 | 1 | 1 | 1 | 1 | 1 |
| | - | Enduring | 1 | 1 | 1 | 1 | 1 | 1 | 1 | 1 | 1 | 1 | 1 | 2 | 1 |
| Shade conformity | Enamel side | | 1 | 1 | 1 | 1 | 1 | 1 | 1 | 1 | 1 | 1 | 1 | 1 | 1 |
| | Dentin side | | 1 | 1 | 1 | 1 | 1 | 1 | 2 | 1 | 1 | 1 | 1 | 1 | 1 |
| Polymerization shrinkage rate | % | | 3.1 | 2.9 | 2.8 | 3.2 | 3.1 | 3.0 | 3.2 | 3.2 | 3.2 | 3.2 | 2.8 | 3.1 | 3.1 |
| Flexural modulus | GPa | Initial | 4.3 | 4.7 | 5 | 3.9 | 4.6 | 4.7 | 4.1 | 4.4 | 4 | 4.8 | 4.1 | 4.7 | 4.3 |
| | GPa | Enduring | 4 | 4.3 | 4.7 | 3.6 | 4.2 | 4.4 | 3.7 | 4.2 | 3.7 | 4.6 | 3.7 | 4.3 | 4 |
| Dissolution | $\mu g/mm^3$ | | 1.0 | 1.0 | 1.0 | 1.0 | 1.0 | 1.0 | 2.0 | 1.0 | 1.0 | 1.2 | 2.0 | 1.2 | 1.2 |

[Table 3]

| Components (parts by mass) | | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 |
|---|---|---|---|---|---|---|---|---|---|---|---|---|---|
| Monomer (a) having acidic group | MDP | 5 | 5 | 5 | 5 | 5 | 5 | 5 | 5 | 10 | | | 10 |
| | GPDM | | | | | | | | | | 5 | | |
| | 4-META | | | | | | | | | | | 5 | |
| Monomer (b) having no acidic group | D-2.6E | 55 | 55 | 55 | 55 | 55 | 55 | 55 | 55 | 50 | 55 | 55 | 40 |
| | Bis-GMA | | | | | | | | | | | | |
| | MAEA | 5 | 5 | 5 | 5 | | 5 | 5 | 5 | 5 | 5 | 5 | 10 |
| | DD | 10 | 20 | | 15 | 5 | | | | | | | 20 |
| | 3G | | | | | | | | | | | | |
| | THF-MA | 15 | 15 | 15 | | 15 | | | | 15 | 15 | 15 | |
| | BEMA | | | | | | 15 | | | | | | |
| | POB-MA | | | | | | | 15 | | | | | |
| | PEMA | | | | | | | | 15 | | | | |
| | HEMA | | | | | | | | | | | | |
| | DEAA | | | | | | | | | | | | |
| | UN7600 | 10 | | | 20 | 20 | 20 | 20 | 20 | 20 | 20 | 20 | 20 |
| | UN7700 | | | 20 | | | | | | | | | |
| Polymerization initiator (c) | CQ | 0.2 | 0.2 | 0.2 | 0.2 | 0.2 | 0.2 | 0.2 | 0.2 | 0.2 | 0.2 | 0.2 | 0.2 |
| | Li-TPO | | | | | | | | | | | | 1 |
| | BAPO-ONa | 0.3 | 0.3 | 0.3 | 0.3 | 0.3 | 0.3 | 0.3 | 0.3 | 0.3 | 0.3 | 0.3 | |
| Filler (d) | Filler 1 | 5 | 5 | 5 | 5 | 5 | 5 | 5 | 5 | 5 | 5 | 5 | |
| | Filler 2 | 40 | 40 | 40 | 40 | 40 | 40 | 40 | 40 | 40 | 40 | 40 | 40 |
| | Filler 3 | 170 | 190 | 170 | 150 | 150 | 150 | 150 | 150 | 150 | 150 | 150 | 130 |
| | Filler 4 | | | | | | | | | | | | |
| | Filler 5 | | | | | | | | | | | | |

(continued)

| Components (parts by mass) | | | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 |
|---|---|---|---|---|---|---|---|---|---|---|---|---|---|---|
| | Filler 6 | | | | | | | | | | | | | |
| | Filler 7 | | | | | | | | | | | | | |
| | Filler 8 | | | | | | | | | | | | | |
| | Filler 9 | | | | | | | | | | | | | |
| | Filler 10 | | | | | | | | | | | | | |
| Polymerization accelerator (e) | DABE | | 0.2 | 0.2 | 0.2 | 0.2 | 0.2 | 0.2 | 0.2 | 0.2 | 0.2 | 0.2 | 0.2 | 0.2 |
| Others | TN326 | | 0.2 | 0.2 | 0.2 | 0.2 | 0.2 | 0.2 | 0.2 | 0.2 | 0.2 | 0.2 | 0.2 | |
| | LBL | | 0.02 | 0.02 | 0.02 | 0.02 | 0.02 | 0.02 | 0.02 | 0.02 | 0.02 | 0.02 | 0.02 | |
| | BHT | | 0.1 | 0.1 | 0.1 | 0.1 | 0.1 | 0.1 | 0.1 | 0.1 | 0.1 | 0.1 | 0.1 | 0.05 |
| | Pigment | | Added | Added | Added | Added | Added | Added | Added | Added | Added | Added | Added | Added |
| Polymerization shrinkage stress | MPa | | 8.2 | 9.3 | 8.6 | 8.2 | 8.2 | 8.4 | 8.2 | 8.6 | 7.2 | 8.8 | 7.9 | 8.3 |
| Degree of light diffusion D of cured product | - | | 0.3 | 0.3 | 0.3 | 0.3 | 0.3 | 0.3 | 0.3 | 0.3 | 0.3 | 0.3 | 0.3 | 0.3 |
| Water absorption | $\mu g/mm^3$ | | 15 | 17 | 12 | 11 | 11 | 10 | 10 | 10 | 14 | 14 | 14 | 15 |
| Shear bond strength (Enamel) | MPa | Initial | 8.6 | 8.5 | 8.4 | 9.2 | 8.6 | 8.4 | 8.4 | 8.4 | 8.4 | 7.8 | 6.4 | 8.6 |
| Shear bond strength (Dentin) | MPa | Initial | 8.2 | 8.4 | 8 | 7.8 | 6.2 | 7.6 | 7.6 | 7.6 | 7.6 | 7 | 6.2 | 7.9 |
| Flexural strength | MPa | Initial | 103 | 110 | 92 | 102 | 90 | 102 | 92 | 106 | 84 | 100 | 88 | 93 |
| Flexural strength | MPa | Enduring | 82 | 94 | 72 | 84 | 71 | 83 | 75 | 88 | 64 | 83 | 68 | 75 |

(continued)

| Components (parts by mass) | | | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 |
|---|---|---|---|---|---|---|---|---|---|---|---|---|---|---|
| Cavity sealing properties | - | Initial | 1 | 1 | 1 | 1 | 1 | 1 | 1 | 1 | 1 | 1 | 1 | 1 |
| | - | Enduring | 1 | 2 | 1 | 1 | 2 | 1 | 1 | 2 | 1 | 1 | 2 | 1 |
| Shade conformity | Enamel side | | 1 | 1 | 1 | 1 | 1 | 1 | 1 | 1 | 1 | 1 | 1 | 1 |
| | Dentin side | | 1 | 1 | 1 | 1 | 1 | 1 | 1 | 1 | 1 | 1 | 1 | 2 |
| Polymerization shrinkage rate | % | | 3.2 | 3.4 | 2.9 | 3.3 | 3.3 | 3.1 | 3.1 | 3.1 | 3.0 | 3.1 | 3.1 | 3.1 |
| Flexural modulus | GPa | Initial | 4.8 | 5.2 | 4.3 | 4.8 | 4.7 | 4.8 | 4.2 | 5.1 | 3.9 | 4.8 | 4 | 4.8 |
| | GPa | Enduring | 4.5 | 4.9 | 3.7 | 4.4 | 4.5 | 4.6 | 3.9 | 4.8 | 3.6 | 4.5 | 3.7 | 4.6 |
| Dissolution | $\mu g/mm^3$ | | 1.1 | 1.0 | 1.0 | 0.9 | 0.9 | 1.0 | 1.0 | 1.0 | 1.2 | 1.2 | 1.2 | 0.8 |

[Table 4]

| Components (parts by mass) | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|
| Monomer (a) having acidic group | MDP | 10 | 10 | 10 | 10 | 5 | 5 | 5 |
| | GPDM | | | | | | | |
| | 4-META | | | | | | | |
| Monomer (b) having no acidic group | D-2.6E | 70 | 45 | | 10 | 75 | 60 | 60 |
| | Bis-GMA | | | | 30 | | | |
| | MAEA | | 10 | | 10 | 5 | 5 | 5 |
| | DD | 20 | 20 | | | | | |
| | 3G | | | 30 | 30 | | 20 | 20 |
| | THF-MA | | | | | 15 | | |
| | BEMA | | | | | | | |
| | POB-MA | | | | | | | |
| | PEMA | | | | | | | |
| | HEMA | | | 30 | 10 | | | |
| | DEAA | | | 30 | | | | |
| | UN7600 | | | | | | 20 | 20 |
| | UN7700 | | 15 | | | | | |
| Polymerization initiator (c) | CQ | 0.2 | 0.2 | 0.2 | 0.2 | 0.2 | 0.2 | 0.2 |
| | Li-TPO | | 1 | | | | | |
| | BAPO-ONa | | | 0.2 | | 0.3 | 0.3 | 0.3 |
| Filler (d) | Filler 1 | | | | | 5 | 5 | |
| | Filler 2 | | | | | 40 | 40 | |
| | Filler 3 | 270 | 170 | 280 | 250 | 150 | 220 | |
| | Filler 4 | | | | | | | |
| | Filler 5 | | | | | | | 180 |
| | Filler 6 | | | | | | | 20 |
| | Filler 7 | | | | | | | |
| | Filler 8 | 10 | 10 | | | | | |
| | Filler 9 | 5 | | | | | | |
| | Filler 10 | | | 20 | | | | |
| Polymerization accelerator (e) | DABE | 0.4 | 0.2 | 0.4 | 0.4 | 0.2 | 0.2 | 0.2 |
| Others | TN326 | | | | | 0.2 | 0.2 | 0.2 |
| | LBL | | | | | 0.02 | 0.02 | 0.02 |
| | BHT | 0.05 | 0.05 | 0.05 | 0.1 | 0.1 | 0.1 | 0.1 |
| | Pigment | | | | | Added | Added | Added |
| Polymerization shrinkage stress | MPa | 10.2 | 8.3 | 13.4 | 11.8 | 10.9 | 10.8 | 11.5 |

(continued)

| Components (parts by mass) | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|
| Degree of light diffusion D of cured product | - | | 0.4 | 0 | 0 | 0 | 0.2 | 0.2 | 0.3 |
| Water absorption | μg/mm³ | | 20 | 15 | 55 | 42 | 18 | 32 | 36 |
| Shear bond strength (Enamel) | MPa | Initial | 5.4 | 10 | 11 | 9.9 | 8.4 | 8.3 | 7.6 |
| Shear bond strength (Dentin) | MPa | Initial | 3.2 | 7.6 | 12.2 | 10.8 | 7.4 | 7.8 | 7 |
| Flexural strength | MPa | Initial | 95 | 92 | 130 | 124 | 115 | 104 | 120 |
| Flexural strength | MPa | Enduring | 86 | 72 | 100 | 99 | 103 | 76 | 106 |
| Cavity sealing properties | - | Initial | 2 | 1 | 2 | 2 | 2 | 2 | 2 |
| | - | Enduring | 3 | 1 | 3 | 3 | 3 | 3 | 3 |
| Shade conformity | Enamel side | | 4 | 4 | 4 | 4 | 1 | 1 | 1 |
| | Dentin side | | 4 | 4 | 4 | 4 | 1 | 1 | 2 |
| Polymerization shrinkage rate | % | | 3.3 | 3 | 3.8 | 3.8 | 3.6 | 3.5 | 3.8 |
| Flexural modulus | GPa | Initial | 104 | 3.8 | 8.7 | 8.9 | 7.2 | 6.4 | 7.0 |
| | GPa | Enduring | 85 | 3.4 | 6.5 | 6.8 | 6.6 | 5.2 | 6.0 |
| Dissolution | μg/mm³ | | 1.1 | 0.8 | 1.5 | 0.9 | 0.8 | 1.2 | 1.1 |

As can be seen from the results presented in Tables 2 and 3, the self-adhesive dental composite resins of Examples had a polymerization shrinkage stress of less than 10 MPa, a degree of light diffusion D of 0.2 or more in the cured product, a water absorption of 20 μg/mm³ or less, a shear bond strength of 5.6 MPa or more to enamel, a shear bond strength of 5.0 MPa or more to dentin, a flexural strength (initial) of 82 MPa or more, a flexural strength (enduring) of 60 MPa or more, a score of 1 for cavity sealing properties (initial), a score of 1 or 2 for cavity sealing properties (enduring), good shade conformity, a polymerization shrinkage rate of 3.4% or less, a flexural modulus (initial) of 3.9 GPa to 5.2 GPa, a flexural modulus (enduring) of 3.6 GPa to 4.9 GPa, and a dissolution of 2.0 μg/mm³ or less.

This is in contrast to Comparative Examples, as shown in Table 4. In Comparative Examples 1 and 3 to 7 with a polymerization shrinkage stress of 10.0 MPa or more, the cavity sealing properties had a score of 2 (initial) and 3 (enduring). In Comparative Examples 2, 3, and 4 in which the cured product had a degree of light diffusion of less than 0.10, the shade conformity was unacceptable both on enamel and dentin sides. The results for Comparative Example 1 appear to be due to the polymer particles (filler 9) failing to sufficiently reduce polymerization shrinkage stress, and the absence of water-soluble photopolymerization initiator or monofunctional hydrophobic monomer, which are effective for adhesive properties to dentin. Comparative Examples 2, 3, and 4 failed to impart light diffusing properties due to the average particle diameter being 0.1 μm or more and the absence of two or more types of fillers with different refractive indices. The compositions of Comparative Examples 3 and 4, which are not formulated to reduce polymerization shrinkage stress, exhibited excellent shear bond strength to tooth structure. However, the cavity sealing properties were poor, presumably due to their notably high polymerization shrinkage stress. Comparative Examples 5, 6, and 7, despite being similar in composition to Examples, showed poor cavity sealing properties, probably as a result of increased polymerization shrinkage stress due to the inclusion of highly curable monomers or the absence of (meth)acrylic compound (b-3).

INDUSTRIAL APPLICABILITY

A self-adhesive dental composite resin of the present invention can be suitably used for filling applications in dental treatment.

Reference Signs List

| | |
|---|---|
| a | Direction of light incidence |
| 1 | Sample plate |
| $L_1$ | Incident light |
| $L_0$ | Transmitted light |
| $I_0$ | Transmitted light at 0-degree angle relative to the direction of light incidence |
| $I_{20}$ | Transmitted light at 20-degree angle relative to the direction of light incidence |
| $I_{70}$ | Transmitted light at 70-degree angle relative to the direction of light incidence |

**Claims**

1. A self-adhesive dental composite resin comprising a monomer (a) having an acidic group, a monomer (b) having no acidic group, a polymerization initiator (c), and a filler (d),

   wherein the self-adhesive dental composite resin has a polymerization shrinkage stress of less than 10.0 MPa, and
   the degree of light diffusion D as indicated by the following formula [I] for the self-adhesive dental composite resin after curing is 0.10 or more and less than 3.00,

$$D = (I_{20}/\cos 20° + I_{70}/\cos 70°)/(2I_0) \qquad [I]$$

   where I represents the luminous intensity of light transmitted through a sample plate made of the self-adhesive dental composite resin after curing, and $I_0$, $I_{20}$, and $I_{70}$ represent the luminous intensity values (intensity of light) at 0-degree, 20-degree, and 70-degree angles, respectively, relative to the direction perpendicular to the sample plate (the direction of light incidence).

2. The self-adhesive dental composite resin according to claim 1, which has a water absorption of less than 40 $\mu g/mm^3$ in its cured product based on ISO4049:2019.

3. The self-adhesive dental composite resin according to claim 1 or 2, which has a shear bond strength of 4.0 MPa or more to dentin based on ISO29022:2013.

4. The self-adhesive dental composite resin according to any one of claims 1 to 3, which has a shear bond strength of 4.0 MPa or more to enamel based on ISO29022:2013.

5. The self-adhesive dental composite resin according to any one of claims 1 to 4, which has a flexural strength of 80 MPa or more in its cured product based on ISO4049:2019.

6. The self-adhesive dental composite resin according to any one of claims 1 to 5, wherein the monomer (b) having no acidic group comprises a monofunctional hydrophobic monomer (b-1a).

7. The self-adhesive dental composite resin according to any one of claims 1 to 6, wherein the filler (d) comprises two or more fillers with different types and/or proportions of constituents.

8. The self-adhesive dental composite resin according to any one of claims 1 to 7, wherein the filler (d) comprises two or more fillers with an average particle diameter of 0.1 μm or more and different refractive indices.

9. The self-adhesive dental composite resin according to claim 8, wherein the fillers (d) with different refractive indices have a refractive index difference of 0.02 or more, and the refractive indices of the fillers (d) with different refractive

indices are 1.40 or more and 1.70 or less.

**10.** The self-adhesive dental composite resin according to claim 8 or 9, wherein one or more of the fillers with different refractive indices comprise at least one metallic element selected from the group consisting of aluminum, strontium, zirconium, barium, lanthanum, ytterbium, titanium, and bismuth.

**11.** The self-adhesive dental composite resin according to any one of claims 1 to 10, wherein the filler (d) comprises a filler (d-i) with an average particle diameter of 1 nm or more and less than 0.1 μm.

**12.** The self-adhesive dental composite resin according to any one of claims 1 to 11, wherein the monomer (a) having an acidic group comprises a monomer having a phosphoric acid group.

**13.** The self-adhesive dental composite resin according to any one of claims 1 to 12, wherein the filler (d) comprises silica-coated ytterbium fluoride.

**14.** The self-adhesive dental composite resin according to any one of claims 1 to 13, wherein the polymerization initiator (c) comprises a water-soluble photopolymerization initiator.

**15.** The self-adhesive dental composite resin according to any one of claims 1 to 14, wherein the monomer (b) having no acidic group comprises a (meth)acrylic compound (b-3) having a weight-average molecular weight of 1,000 or more and less than 80,000, and a weight-average molecular weight of 1,250 or more and less than 20,000 per polymerizable group.

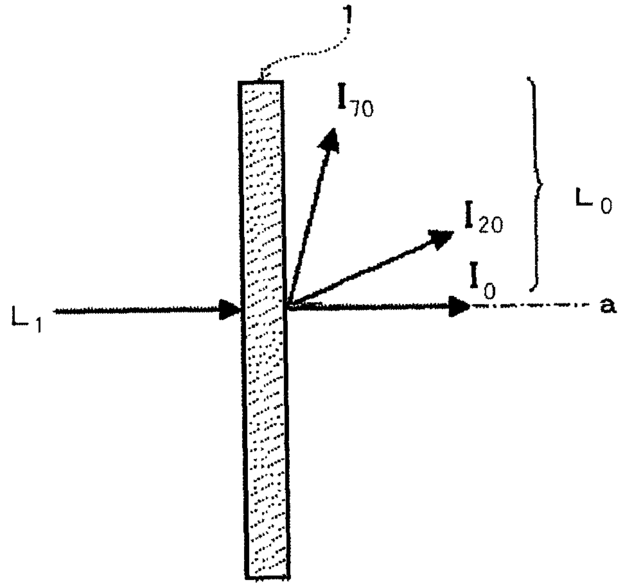
1
$I_{70}$
$I_{20}$
$I_0$
$L_0$
$L_1$
a

FIG.1

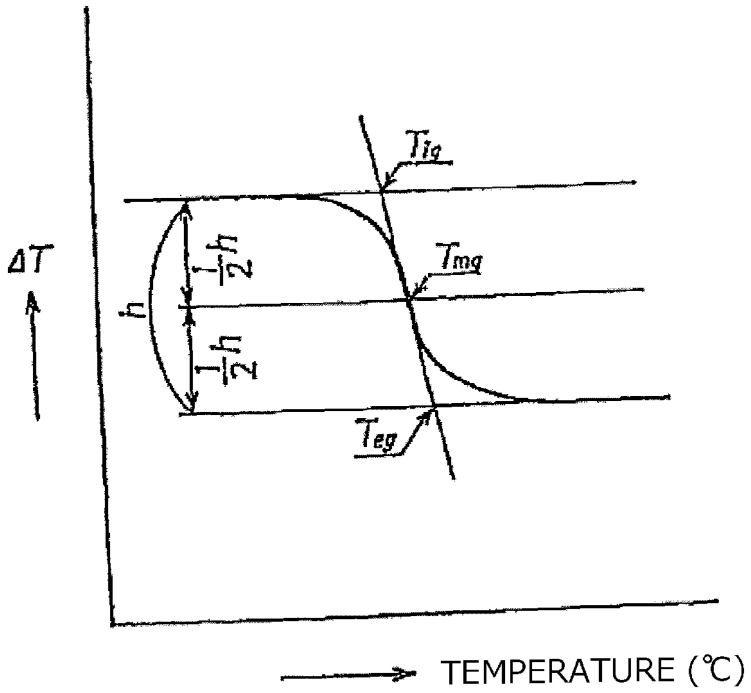
$T_{ig}$
$T_{mg}$
$T_{eg}$
$\Delta T$
h
$\frac{1}{2}h$
$\frac{1}{2}h$
TEMPERATURE (℃)

FIG.2

**INTERNATIONAL SEARCH REPORT**

International application No.
**PCT/JP2023/013749**

**A. CLASSIFICATION OF SUBJECT MATTER**

***A61K 6/887***(2020.01)i; ***A61K 6/16***(2020.01)i; ***A61K 6/17***(2020.01)i; ***A61K 6/62***(2020.01)i; ***A61K 6/831***(2020.01)i; ***A61K 6/84***(2020.01)i; ***A61K 6/842***(2020.01)i; ***A61K 6/893***(2020.01)i
FI: A61K6/887; A61K6/16; A61K6/17; A61K6/62; A61K6/84; A61K6/842; A61K6/893; A61K6/831

According to International Patent Classification (IPC) or to both national classification and IPC

**B. FIELDS SEARCHED**

Minimum documentation searched (classification system followed by classification symbols)

A61K6/887; A61K6/16; A61K6/17; A61K6/62; A61K6/831; A61K6/84; A61K6/842; A61K6/893

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2021/070875 A1 (KURARAY NORITAKE DENTAL INC.) 15 April 2021 (2021-04-15) paragraphs [0016], [0097]-[0107], [0129]-[0137], claims, examples 1-8 | 1-15 |
| Y | JP 2002-138008 A (TOKUYAMA CORP.) 14 May 2002 (2002-05-14) paragraphs [0002]-[0012], [0075], examples, claims | 1-15 |
| Y | WO 2015/125470 A1 (KURARAY NORITAKE DENTAL INC.) 27 August 2015 (2015-08-27) paragraphs [0006]-[0012], [0083]-[0086], examples, claims | 1-15 |
| Y | WO 2021/054457 A1 (KURARAY NORITAKE DENTAL INC.) 25 March 2021 (2021-03-25) paragraphs [0006]-[0008], [0013]-[0027], examples, claims | 1-15 |
| Y | JP 2017-524020 A (IVOCLAR VIVADENT AG) 24 August 2017 (2017-08-24) paragraphs [0010], [0016]-[0020], examples, claims | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 June 2023** | **27 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/013749**

**C. DOCUMENTS CONSIDERED TO BE RELEVANT**

| Category* | Citation of document, with indication, where appropriate, of the relevant passages | Relevant to claim No. |
|---|---|---|
| Y | JP 2018-145133 A (KURARAY NORITAKE DENTAL INC.) 20 September 2018 (2018-09-20)<br>paragraphs [0147], [0165], examples | 1-15 |
| A | WO 2021/117839 A1 (KURARAY NORITAKE DENTAL INC.) 17 June 2021 (2021-06-17)<br>entire text | 1-15 |

Form PCT/ISA/210 (second sheet) (January 2015)

**INTERNATIONAL SEARCH REPORT**
**Information on patent family members**

International application No.
**PCT/JP2023/013749**

| Patent document cited in search report | Publication date (day/month/year) | Patent family member(s) | Publication date (day/month/year) |
|---|---|---|---|
| WO 2021/070875 A1 | 15 April 2021 | EP 4043002 A1<br>paragraphs [0016], [0101]-[0111], [0136]-[0142], claims, examples 1-8 | |
| JP 2002-138008 A | 14 May 2002 | US 2003/0036582 A1<br>paragraphs [0002]-[0017], [0165]-[0168], examples, claims<br>EP 1226807 A1 | |
| WO 2015/125470 A1 | 27 August 2015 | US 2017/0049665 A1<br>paragraphs [0006]-[0019], [0091]-[0095], examples, claims<br>EP 3108868 A1 | |
| WO 2021/054457 A1 | 25 March 2021 | US 2022/0362110 A1<br>paragraphs [0006]-[0008], [0023]-[0041], examples, claims<br>EP 4032522 A1 | |
| JP 2017-524020 A | 24 August 2017 | US 2017/0224591 A1<br>paragraphs [0009], [0016]-[0023], examples, claims<br>EP 2987480 A1<br>CN 106659640 A | |
| JP 2018-145133 A | 20 September 2018 | (Family: none) | |
| WO 2021/117839 A1 | 17 June 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004529946 T
- JP 2017105716 A
- JP 2018065831 A
- WO 2019044815 A
- WO 2015190101 A
- WO 2021117839 A
- WO 2021070875 A
- JP S57197289 A
- WO 2014095724 A
- JP H093109 A
- JP H10245525 A
- WO 2008087977 A


### Non-patent literature cited in the description


- **SUH, CLARKE**. *J. P. S. A-1*, 1967, vol. 5, 1671-1681
- **FEDORS et al.** Polymer Engineering and Science. *ROBERF. FEDORS.*, February 1974, vol. 14 (2), 147-154